# EUROPEAN PATENT APPLICATION

(11) **EP 2 572 709 A1**
(43) Date of publication of application: **27.03.2013**
(21) Application number: 12198383.7
(22) Date of filing: 25.11.2009
(51) Int. Cl.: A61K 31/00, A61K 31/519, A61P 35/00

(54) **HSP90 inhibitors for therapeutic treatment**

(30) Priority: 28.11.2008 EP 08170284
(62) Divisional of application: 09756530.3
(71) Applicant: Novartis AG, 4056 Basel (CH)
(72) Inventor: Jensen, Michael Rugaard, 4002 Basel (CH); Quadt, Cornelia, 4002 Basel (CH); Garcia-Echeverria, Carlos, 92210 Saint-Cloud (FR)
(74) Representative: Rudge, Sewkian

(57) **Abstract**

The use of a Hsp90 inhibitor for the treatment of cancer of the lung, wherein the lung cancer is selected from non small cell lung cancer, small cell lung cancer and pleural mesothelioma.

## Description

This application claims priority to European Application Number 08170284.7, filed November 28, 2008, the contents of which are incorporated by reference.

### BACKGROUND OF THE INVENTION

### Field of the Invention

The invention relates to the use of a Hsp 90 inhibitor for the manufacture of pharmaceutical compositions for use in the treatment of cancer.

### Related Background Art

Heat shock protein 90 (Hsp90) is recognized as a new anti-cancer target. Hsp90 is a ubiquitous, highly abundant (1-2% of the total cellular protein), essential protein which functions as a molecular chaperone to ensure the conformational stability, shape and function of client proteins. Inhibition of its intrinsic ATPase activity of Hsp90 disrupts the Hsp90-client protein interaction resulting in their degradation via the ubiquitin proteasome pathway. A subset of Hsp90 client proteins, such as Raf, AKT, CDK4 and the EGFR family including ErbB2 are oncogenic signaling molecules critically involved in cell growth, differentiation and apoptosis, processes which are fundamentaly important in cancer cells. The simultaneous degradation of multiple oncoproteins is believed to produce the antitumor effects observed with Hsp90 inhibitors.

The Hsp90 family of chaperones is comprised of four members: Hsp90α and Hsp90β both located in the cytosol, GRP94 in the endoplasmic reticulum, and TRAP1 in the mitochondria (Csermely et al., 1998). Hsp90 is the most abundant cellular chaperone, constituting about 1% - 2% of total protein (Jakob and Buchner, 1994). Among the stress proteins, Hsp90 is unique because it is not required for the biogenesis of most polypeptides (Nathan et al., 1997). Its cellular targets, also called client proteins, are conformationally labile signal transducers that play a critical role in growth control, cell survival and tissue development (Pratt and Toft, 2003).

Hsp90 chaperones, which possess a conserved ATP-binding site at their N-terminal domain (Chene, 2002) belong to a small ATPase sub-family known as the DNA Gyrase, Hsp90, Histidine Kinase and MutL (GHKL) sub-family (Dutta and Inouye, 2000). The chaperoning (folding) activity of Hsp90 depends on its ATPase activity which is weak for the isolated enzyme. However, it has been shown that the ATPase activity of Hsp90 is enhanced upon its association with proteins known as co-chaperones (Kamal et al., 2003). Therefore, in vivo, Hsp90 proteins work as subunits of large, dynamic protein complexes. Hsp90 is essential for eukaryotic cell survival and is overexpressed in many tumors.

### BRIEF SUMMARY OF THE INVENTION

In invention relates to treatment of proliferate diseases such as cancer with a compound according to Formula (I) 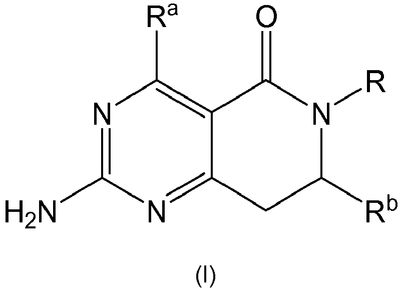 or a stereoisomer, tautomer, pharmaceutically acceptable salt, or prodrug thereof, wherein
R^{a} is selected from the group consisting of
(1) hydrogen,
(2) halogen,
(3) hydroxyl,
(4) C₁-C₆ alkoxy,
(5) thiol,
(6) C₁-C₆ alkylthiol,
(7) substituted or unsubstituted C₁-C₆ alkyl,
(8) amino or substituted amino,
(9) substituted or unsubstituted aryl,
(10) substituted or unsubstituted heteroaryl, and
(11) substituted or unsubstituted heterocyclyl;
R is selected from the group consisting of
(1) hydrogen,
(2) substituted or unsubstituted C₁-C₆ alkyl,
(3) substituted or unsubstituted C₂-C₆ alkenyl,
(4) substituted or unsubstituted C₂-C₆ alkynyl,
(5) substituted or unsubstituted C₃-C₇ cycloalkyl,
(6) substituted or unsubstituted C₅-C₇ cycloalkenyl,
(7) substituted or unsubstituted aryl,
(8) substituted or unsubstituted heteroaryl, and
(9) substituted or unsubstituted heterocyclyl;
R^{b} is selected from the group consisting of
(1) substituted or unsubstituted C₃-C₇ cycloalkyl,
(2) substituted or unsubstituted C₅-C₇ cycloalkenyl,
(3) substituted or unsubstituted aryl,
(4) substituted or unsubstituted heteroaryl, and
(5) substituted or unsubstituted heterocyclyl; and
with the proviso that when R^{a} is amino, then R^{b} is not phenyl, 4-alkyl-phenyl, 4-alkoxy-phenyl, or 4-halo-phenyl.

### DETAILED DESCRIPTION OF THE INVENTION

The Hsp90 inhibitor compounds of the present invention are useful in the treatment of cancer, e.g. solid tumors, e.g. sarcomas, e.g. carcinomas of the bladder, the colon, the rectum, the liver, the lung, e.g. pleural mesothelioma, e.g. non small cell, e.g. small cell, the breast, the vagina, the ovaries, the uterus, the pancreas, the kidney, the stomach, gastrointestinal tract, e.g. gastrointestinal stromal tumor, e.g. the small intestine, e.g. the esophagus, e.g. the bile duct, the prostate, the testis, the head and neck, the peritoneal cavity, the thyroid, the bone, the brain, the central nervous system e.g. glioblastoma, e.g. neuroblastoma, neurofibromatosis, chordoma, thymoma, adenoid cystic carcinoma, aggressive fibromatosis, myelofibrosis, desmoplastic small cell round tumor, and/or the skin, e.g. melanoma and/or the endocrine system, e.g. the thyroid, the adrenal gland, and/or cancer of the blood , e.g. hematological cancer, e.g. leukemia, e.g. acute myeloid leukemia, e.g. chronic myeloid leukemia, e.g. chronic lymphatic leukemia, e.g. acute lymphatic leukemia, e.g. multiple myeloma e.g. lymphomas, and/or for use in treatment of myelodysplastic syndrome, systemic mastocytosis, von Hippel-Lindau syndrome, multicentric Castleman disease and/or psioriasis, to the use of a Hsp90 inhibitor in the treatment of cancer, e.g. solid tumors, e.g. sarcomas, e.g. carcinomas of the bladder, the colon the rectum, the liver, the lung, e.g. pleural mesothelioma, e.g. non small cell, e.g. small cell, the breast, the vagina, the ovaries, the uterus, the pancreas, the kidney, the stomach, gastrointestinal tract, e.g. gastrointestinal stromal tumor, e.g. the small intestine, e.g. the esophagus, e.g. the bile duct, the prostate, the testis, the head and neck, the peritoneal cavity, the thyroid, the bone, the brain, the central nervous system e.g. glioblastoma, e.g. neuroblastoma, neurofibromatosis, chordoma, thymoma, adenoid cystic carcinoma, aggressive fibromatosis, myelofibrosis, desmoplastic small cell round tumor and/or the skin, e.g. melanoma and/or the endocrine system, e.g. the thyroid, the adrenal gland, and/or cancer of the blood , e.g. hematological cancer, e.g. leukemia, e.g. acute myeloid leukemia, e.g. chronic myeloid leukemia, e.g. chronic lymphatic leukemia, e.g. acute lymphatic leukemia, e.g. multiple myeloma e.g. lymphomas, and/or in the treatment of myelodysplastic syndrome, systemic mastocytosis, von Hippel-Lindau syndrome, multicentric Castleman disease and/or psioriasis, and to a method of treating warm-blooded animals including humans suffering from cancer, e.g. solid tumors, e.g. sarcomas, e.g. carcinomas of the bladder, the colon, the rectum, the liver, the lung, e.g. pleural mesothelioma, e.g. non small cell, e.g. small cell, the breast, the vagina, the ovaries, the uterus, the pancreas, the kidney, the stomach, gastrointestinal tract, e.g. gastrointestinal stromal tumor, e.g. the small intestine, e.g. the esophagus, e.g. the bile duct, the prostate, the testis, the head and neck, the peritoneal cavity, the thyroid, the bone, the brain, the central nervous system e.g. glioblastoma, e.g. neuroblastoma, neurofibromatosis, chordoma, thymoma, adenoid cystic carcinoma, aggressive fibromatosis, myelofibrosis, desmoplastic small cell round tumor and/or the skin, e.g. melanoma and/or the endocrine system, e.g. the thyroid, the adrenal gland,and/or cancer of the blood , e.g. hematological cancer, e.g. leukemia, e.g. acute myeloid leukemia, e.g. chronic myeloid leukemia, e.g. chronic lymphatic leukemia, e.g. acute lymphatic leukemia, e.g. multiple myeloma e.g. lymphomas, and/or myelodysplastic syndrome, systemic mastocytosis, von Hippel-Lindau syndrome, multicentric Castleman disease and/or psioriasis by administering to said animal in need of such treatment an effective dose of a Hsp 90 inhibitor. Management of cancer, e.g. solid tumors, e.g. sarcomas, e.g. carcinomas of the bladder, the colon, the rectum, the liver, the lung, e.g. pleural mesothelioma, e.g. non small cell, e.g. small cell, the breast, the vagina, the ovaries, the uterus, the pancreas, the kidney, the stomach, gastrointestinal tract, e.g. gastrointestinal stromal tumor, e.g. the small intestine, e.g. the esophagus, e.g. the bile duct, the prostate, the testis, the head and neck, the peritoneal cavity, the thyroid, the bone, the brain, the central nervous system e.g. glioblastoma, e.g. neuroblastoma, neurofibromatosis, chordoma, thymoma, adenoid cystic carcinoma, aggressive fibromatosis, myelofibrosis, desmoplastic small cell round tumor and/or the skin, e.g. melanoma and/or the endocrine system, e.g. the thyroid, the adrenal gland,and/or cancer of the blood , e.g. hematological cancer, e.g. leukemia, e.g. acute myeloid leukemia, e.g. chronic myeloid leukemia, e.g. chronic lymphatic leukemia, e.g. acute lymphatic leukemia, e.g. multiple myeloma e.g. lymphomas, and/or myelodysplastic syndrome, systemic mastocytosis, von Hippel-Lindau syndrome, multicentric Castleman disease and/or psioriasis is a major problem.

In particular embodiments, the Hsp90 component is a compound according to formula (Ia) 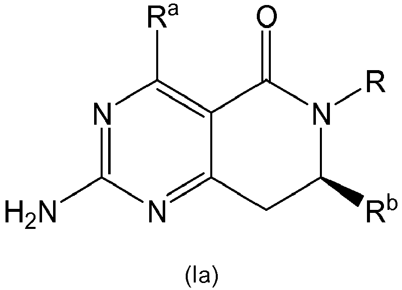 or a tautomer, pharmaceutically acceptable salt, or prodrug thereof, wherein R, R^{a}, and R^{b} are as previously defined for Formula (I) and with the proviso that when R^{a} is amino, then R^{b} is not phenyl, 4-alkyl-phenyl, 4-alkoxy-phenyl, or 4-halo-phenyl. In some embodiments of the compounds of Formula (I) or (Ia), R^{a} is hydrogen.

In other embodiments, R^{a} is substituted or unsubstituted C₁-C₆ alkyl.

In some embodiments, R^{a} is C₁-C₆ alkyl or halo C₁-C₆ alkyl. In some such embodiments, R^{a} is methyl.

In some embodiments, R^{b} is aryl or heteroaryl. In some such embodiments, R^{b} is selected from the group consisting of phenyl, pyridyl, pyrimidinyl, pyrazinyl, indolyl, thiazolyl, and thienyl, each of which can be substituted or unsubstituted. In some aspects, the invention provides compounds wherein the aforementioned R^{b} groups are substituted with substituted or unsubstituted aryl, or substituted or unsubstituted heteroaryl. In other aspects the R^{b} groups are substituted with halo. In still other aspects the R^{b} groups are substituted with fluoro. In still other aspects, the R^{b} groups are substituted with alkyl, haloalkyl, alkoxy, and haloalkoxy. In some aspects, the R^{b} groups are substituted with methyl. In other aspects, the R^{b} groups are substituted with methoxy.

In other embodiments, R^{b} is selected from the group consisting of substituted aryl, substituted heterocyclyl, substituted heteroaryl, substituted C₃-C₇ cycloalkyl, and substituted C₅-C₇ cycloalkenyl, wherein said aryl, heterocyclyl, heteroaryl, C₃-C₇ cycloalkyl, and C₅-C₇ cycloalkenyl is selected from the group consisting of pyrrolyl, phenyl, pyridyl, pyrazinyl, pyrimidinyl, pyridazinyl, pyrazolyl, imidazolyl, triazolyl, indolyl, oxadiazole, thiadiazole, furanyl, quinolinyl, isoquinolinyl, isoxazolyl, oxazolyl, thiazolyl, morpholino, piperidinyl, pyrrolidinyl, thienyl, cyclohexyl, cyclopentyl, cyclohexenyl, and cyclopentenyl. In some aspects, the aforementioned groups are substituted with one to two substituents selected from the group consisting of halo, alkoxy, alkyl, amino, alkylamino, haloalkyl, and haloalkoxy.

In some embodiments, R is selected from the group consisting of hydrogen, unsubstituted alkyl, and substituted alkyl. In some such embodiments, R is selected from the group consisting of methyl, ethyl, allyl, 3-methyl-butyl, and isobutyl. In other embodiments, R is selected from the group consisting of hydrogen, benzyl, 1-(4-methoxyphenyl)ethyl, methyl, 3-aminopropyl, and 2-methyl-2-morpholinopropyl. In still other embodiment, R is hydrogen. In another embodiment, the 2-amino-7,8-dihydro-6H-pyrido[4,3-d]pyrimidin-5-one compounds have the formula (II): 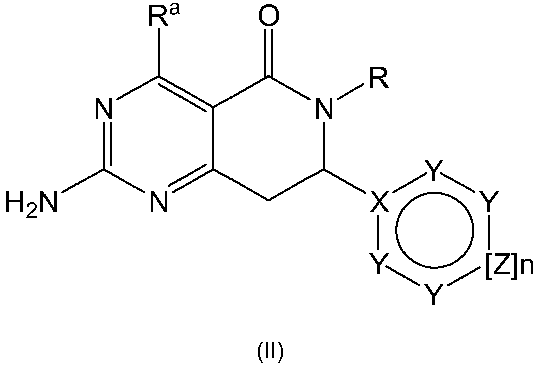 or a stereoisomer, tautomer, pharmaceutically acceptable salt, or prodrug thereof, wherein
n is 0 or 1,
wherein R^{a} is selected from the group consisting of
(1) hydrogen,
(2) halogen,
(3) hydroxyl,
(4) C₁-C₆ alkoxy,
(5) thiol,
(6) C₁-C₆ alkylthiol,
(7) substituted or unsubstituted C₁-C₆ alkyl,
(8) amino or substituted amino,
(9) substituted or unsubstituted aryl,
(10) substituted or unsubstituted heteroaryl, and
(11) substituted or unsubstituted heterocyclyl;
wherein R is selected from the group consisting of
(1) hydrogen,
(2) substituted or unsubstituted C₁-C₆ alkyl,
(3) substituted or unsubstituted C₂-C₆ alkenyl,
(4) substituted or unsubstituted C₂-C₆ alkynyl,
(5) substituted or unsubstituted C₃-C₇ cycloalkyl,
(6) substituted or unsubstituted C₅-C₇ cycloalkenyl,
(7) substituted or unsubstituted aryl,
(8) substituted or unsubstituted heteroaryl, and
(9) substituted or unsubstituted heterocyclyl,
wherein when n is 1, X is C, Y is at each position independently selected from CQ¹ and N, and Z is selected from CR² and N with the proviso that no more than 3 Y and Z groups are N, and
wherein when n is 0, X is C or N, Y is at each position independently selected from CQ¹, N, NQ², O, and S with the proviso that no more than 4 X and Y groups are N and NQ² and no more than 1 Y group is S or O;
wherein Q¹ is at each position independently selected from the group consisting of
(1) hydrogen,
(2) halogen,
(3) substituted or unsubstituted C₁-C₆ alkyl,
(4) substituted or unsubstituted C₂-C₆ alkenyl,
(5) substituted or unsubstituted C₂-C₆ alkynyl,
(6) substituted or unsubstituted C₃-C₇ cycloalkyl,
(7) substituted or unsubstituted C₅-C₇ cycloalkenyl,
(8) substituted or unsubstituted aryl,
(9) substituted or unsubstituted heteroaryl,
(10) substituted or unsubstituted heterocyclyl,
(11) substituted or unsubstituted amino,
(12) -OR³ or -SR³,
(13) -C(O)R³, -CO₂R³, -C(O)N(R³)₂, -S(O)R³, -SO₂R³, or -SO₂N(R³)₂,
(14) -OC(O)R³, -N(R³)C(O)R³, or -N(R³)SO₂R³,
(15) -CN, and
(16) -NO₂;
wherein Q² is at each position independently selected from the group consisting of
(1) hydrogen,
(3) substituted or unsubstituted C₁-C₆ alkyl,
(4) substituted or unsubstituted C₂-C₆ alkenyl,
(5) substituted or unsubstituted C₂-C₆ alkynyl,
(6) substituted or unsubstituted C₃-C₇ cycloalkyl,
(7) substituted or unsubstituted C₅-C₇ cycloalkenyl,
(8) substituted or unsubstituted aryl,
(9) substituted or unsubstituted heteroaryl, and
(10) substituted or unsubstituted heterocyclyl;
wherein R² is selected from the group consisting of
(1) hydrogen,
(2) halogen,
(3) substituted or unsubstituted C₁-C₃ alkyl, and
(4) -OR³, -SR³, or-NHR³;
wherein R³ is at each position independently selected from the group consisting of
(1) hydrogen,
(2) substituted or unsubstituted C₁-C₆ alkyl,
(3) substituted or unsubstituted C₂-C₆ alkenyl,
(4) substituted or unsubstituted C₂-C₆ alkynyl,
(5) substituted or unsubstituted C₃-C₇ cycloalkyl,
(6) substituted or unsubstituted C₅-C₇ cycloalkenyl,
(7) substituted or unsubstituted aryl,
(8) substituted or unsubstituted heteroaryl, and
(9) substituted or unsubstituted heterocyclyl,
with the proviso that when R^{a} is amino, then X, Y, Z, and n together do not form a phenyl, 4-alkyl-phenyl, 4-alkoxy-phenyl, or 4-halo-phenyl group.

In other embodiments, the first pharmaceutical component of the invention is described according to formula (IIa): 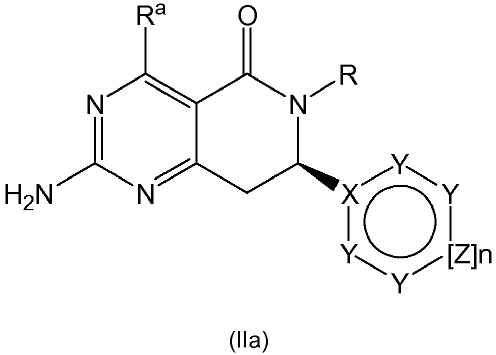 or a tautomer, pharmaceutically acceptable salt, or prodrug thereof, wherein R^{a}, R, X, Y, Z, and n are previously defined for formula (II) and with the proviso that when R^{a} is amino, then X, Y, Z, and n together do not form a phenyl, 4-alkyl-phenyl, 4-alkoxy-phenyl, or 4-halo-phenyl group.

In some embodiments when n is 0, X is C, and Y adjacent to X is not O.

In some embodiments of the compounds of formula (II) or (IIa), R^{a} is hydrogen.

In other embodiments, R^{a} is substituted or unsubstituted C₁-C₆ alkyl.

In some embodiments, R^{a} is C₁-C₆ alkyl or halo C₁-C₆ alkyl. In some such embodiments, R^{a} is methyl.

For the compounds of Formula (I), (Ia), (II), or (IIa), representative substituted alkyl groups include arylalkyl, heteroarylalkyl, cycloalkylalkyl, heterocyclylalkyl, aminoalkyl, alkylaminoalkyl, dialkylaminoalkyl, and sulfonamidoalkyl groups.

Representative aryl groups include phenyl groups.

Representative heteroaryl groups include pyridyl, pyrazinyl, pyrimidinyl, pyridazinyl, pyrazolyl, indolyl, quinolinyl, isoquinolinyl, furanyl, oxazolyl, thiazolyl, and thienyl groups. In one embodiment, one of Q¹ or Q² is selected from the group consisting of substituted and unsubstituted phenyl, substituted and unsubstituted pyridyl, substituted and unsubstituted pyrimidinyl, substituted and unsubstituted pyrazinyl, substituted and unsubstituted indolyl, substituted and unsubstituted thiazolyl, and substituted and unsubstituted thienyl. In one embodiment, one of Q¹ or Q² is selected from the group consisting of piperidinyl, morpholinyl, pyrrolidinonyl, and benzyl amino.

In one embodiment, one of Q¹ or Q² is selected from the group consisting of cyclohexyl and cyclopentyl.

In one embodiment, one of Q¹ or Q² is selected from the group consisting of cyclohexenyl and cyclopentenyl.

In one embodiment, one of Q¹ or Q² is selected from the group consisting of substituted aryl, substituted heterocyclyl, substituted heteroaryl, substituted C₃-C₇ cycloalkyl, and substituted C₅-C₇ cycloalkenyl, wherein said aryl, heterocyclyl, heteroaryl, C₃-C₇ cycloalkyl, and C₅-C₇ cycloalkenyl is selected from the group consisting of pyrrolyl, phenyl, pyridyl, pyrazinyl, pyrimidinyl, pyridazinyl, pyrazolyl, imidazolyl, triazolyl, indolyl, oxadiazole, thiadiazole, furanyl, quinolinyl, isoquinolinyl, isoxazolyl, oxazolyl, thiazolyl, morpholino, piperidinyl, pyrrolidinyl, thienyl, cyclohexyl, cyclopentyl, cyclohexenyl, and cyclopentenyl. In some aspects, the aforementioned groups are substituted with one to two substituents selected from the group consisting of halo, alkoxy, alkyl, amino, alkylamino, haloalkyl, and haloalkoxy.

In one embodiment, one of Q¹ or Q² is selected from substituted and unsubstituted pyridyl, substituted and unsubstituted pyrazinyl, substituted and unsubstituted phenyl, substituted and unsubstituted isoquinolinyl, substituted and unsubstituted pyrimidinyl, substituted and unsubstituted pyrazolyl, and substituted and unsubstituted furanyl. In some aspects, the aforementioned groups are substituted with one to two substituents selected from the group consisting of halo, alkoxy, alkyl, amino, alkylamino, haloalkyl, and haloalkoxy.

In other embodiments one of Q¹ or Q² is selected from the group consisting of (2-hydroxy-ethylamino)-pyrazin-2-yl, 1H-pyrazol-4-yl, 1-methyl-1H-pyrazol-4-yl, 1-methyl-1H-pyrazol-4-yl, 2-(5-methyl-pyridin-2-yl)-phenyl, 2,3-difluoro-phenyl, 2,3-dimethoxy-phenyl, 2,4-difluoro-phenyl, 2,4-dimethoxy-phenyl, 2,4-dimethoxy-pyrimidin-5-yl, 2,5-difluoro-phenyl, 2,6-difluoro-phenyl, 2,6-dimethyl-pyridin-3-yl, 2-acetamidophenyl, 2-aminocarbonylphenyl, 2-amino-pyrimidin-5-yl, 2-chloro-4-methoxy-pyrimidin-5-yl, 2-chloro-5-fluoro-pyridin-3-yl, 2-chloro-phenyl, 2-chloro-pyridin-3-yl, 2-chloro-pyridin-4-yl, 2-difluoro-3-methoxyphenyl, 2-ethyl-phenyl, 2-ethoxy-thiazol-4-yl, 2-fluoro-3-methoxy-phenyl, 2-fluoro-3-methylphenyl, 2-fluoro-4-methyl-phenyl, 2-fluoro-5-methoxy-phenyl, 2-fluoro-5-methylphenyl, 2-fluorophenyl, 2-fluoro-pyridin-3-yl, 2-hydroxymethyl-3-methoxyphenyl, 2-hydroxymethylphenyl, 2-isoquinolin-4-yl, 2-methoxy-5-trifluoromethyl-phenyl, 2-methoxy-phenyl, 2-methoxy-pyridin-3-yl, 2-methoxy-pyrimidin-4-yl, 2-methoxy-thiazol-4-yl, 2-methyl-phenyl, 2-methyl-pyridin-3-yl, 2-oxo-1,2-dihydro-pyridin-3-yl, 2-phenoxyphenyl, 2-pyridin-3-yl, 2-pyrimidin-5-yl, 2-trifluoromethoxyphenyl, 2-trifluoromethoxy-phenyl, 3,4-dimethoxy-phenyl, 3,5-dimethyl-isoxazol-4-yl, 3,6-dimethyl-pyrazin-2-yl, 3-acetamidophenyl, 3-aminocarbonylphenyl, 3-bromo-phenyl, 3-chloro-pyrazin-2-yl, 3-cyanophenyl, 3-dimethylaminophenyl, 3-ethoxyphenyl, 3-ethyl-4-methyl-phenyl, 3-ethynyl-phenyl, 3-fluoro-6-methoxy-pyridin-2-yl, 3-fluorophenyl, 3-fluoro-pyrazin-2-yl, 3-methanesulfonamidophenyl, 3-methoxycarbonylphenyl, 3-methoxyphenyl, 3-methoxy-pyrazin-2-yl, 3-methyl-3H-imidazo[4,5-b]pyrazin-5-yl, 3-methylphenyl, 3-methyl-pyridin-2-yl, 3-trifluoromethoxyphenyl, 3-trifluoromethylphenyl, 4,5-dimethoxy-pyrimidin-2-yl, 4-amino-5-fluoro-pyrimidin-2-yl, 4-chloro-2,5-dimethoxy-phenyl, 4-chloro-2-fluoro-phenyl, 4-chloro-2-methoxy-5-methyl-phenyl, 4-chloro-pyridin-3-yl, 4-difluoro-2-methyl-phenyl, 4-ethoxy-5-fluoro-pyrimidin-2-yl, 4-ethoxy-pyrimidin-2-yl, 4-ethoxy-pyrimidin-5-yl, 4-ethyl-1 H-pyrazol-3-yl, 4-fluoro-2-methoxy-phenyl, 4-fluoro-2-methyl-phenyl, 4-fluorophenyl, 4-methoxy-5-methyl-pyrimidin-2-yl, 4-methoxy-pyridin-3-yl, 4-methoxy-pyrimidin-2-yl, 4-methoxy-pyrimidin-5-yl, 4-methyl-phenyl, 4-methyl-pyridin-2-yl, 4-methyl-pyridin-3-yl, 4-pyrrolidin-1-yl-pyrimidin-2-yl, 5,6-dimethoxy-pyrazin-2-yl, 5-acetyl-thiophen-2-yl, 5-amino-6-ethoxy-pyrazin-2-yl, 5-amino-6-methoxy-3-methyl-pyrazin-2-yl, 5-amino-6-methoxy-pyridin-2-yl, 5-chloro-4-methoxy-pyrimidin-2-yl, 5-chloro-6-methoxy-pyrazin-2-yl, 5-dimethylamino-6-methoxy-pyrazin-2-yl, 5-fluoro-2-methoxyphenyl, 5-fluoro-4-methoxy-pyrimidin-2-yl, 5-fluoro-6-methoxy-pyrazin-2-yl, 5-fluoro-pyridin-2-yl, 5-methoxy-pyridin-3-yl, 5-methoxy-thiophen-2-yl, 5-trifluoromethyl-pyrimidin-2-yl, 6-acetyl-pyridin-2-yl, 6-chloro-pyrazin-2-yl, 6-ethoxy-pyrazin-2-yl, 6-ethoxy-pyridin-2-yl, 6-fluoro-pyridin-2-yl, 6-fluoro-pyridin-3-yl, 6-hydroxy-pyridin-2-yl, 6-methoxy-5-methylamino-pyrazin-2-yl, 6-methoxy-5-methyl-pyrazin-2-yl, 6-methoxy-pyrazin-2-yl, 6-methoxy-pyridin-2-yl, 6-methoxy-pyridin-3-yl, 6-methylamino-pyrazin-2-yl, 6-methyl-pyridin-2-yl, 5-amino-6-(2,2,2-trifluoroethoxy)pyrazin-2-yl, and 6-trifluoromethyl-pyridin-2-yl.

In one embodiment Q¹ is halo.

In one embodiment Q¹ is alkyl. In some aspects, Q¹ is methyl.

In one embodiment, R² is selected from hydrogen and fluoro. In some aspects, R² is fluoro.

In one embodiment, R² is selected from alkyl. In some aspects, R² is methyl.

In one embodiment, R² is selected from alkoxy. In some aspects, R² is methoxy.

In one embodiment Q¹ is OR³.

In one embodiment, R³ is selected from the group consisting of methyl, ethyl, isopropyl, cyclopentyl, and cyclohexyl.

In one embodiment, R³ is selected from substituted and unsubstituted phenyl, substituted and unsubstituted thiazolyl, substituted and unsubstituted pyridyl, substituted and unsubstituted pyrazinyl, and substituted and unsubstituted pyrimidinyl. In one embodiment, R³ is selected from the group consisting of 2-aminoethyl, 2-piperidinylethyl, 2-piperazinylethyl, 2-morpholinylethyl, and 2-(N-methylpiperazinyl)ethyl. In some embodiments, R is selected from the group consisting of hydrogen, unsubstituted alkyl, and substituted alkyl. In some such embodiments, R is selected from the group consisting of methyl, ethyl, allyl, 3-methyl-butyl, and isobutyl. In other embodiments, R is selected from the group consisting of hydrogen, benzyl, 1-(4-methoxyphenyl)ethyl, methyl, 3-aminopropyl, and 2-methyl-2-morpholinopropyl.

In another embodiment of the invention, compounds of formula (III) are provided as the first component, in combination with a HER-2 inhibitor as the second component: 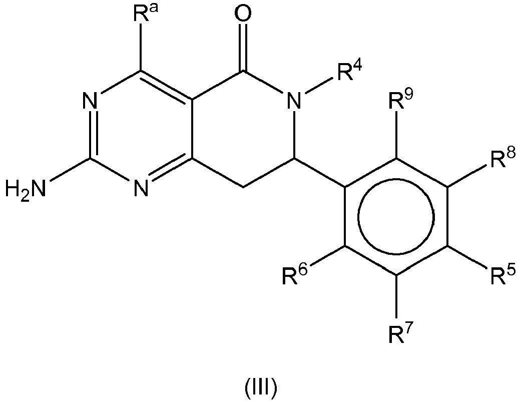 or a stereoisomer, tautomer, pharmaceutically acceptable salt, or prodrug thereof, wherein
wherein R^{a} is selected from the group consisting of
(1) hydrogen,
(2) halogen,
(3) hydroxyl,
(4) C₁-C₆ alkoxy,
(5) thiol,
(6) C₁-C₆ alkylthiol,
(7) substituted or unsubstituted C₁-C₆ alkyl,
(8) amino or substituted amino,
(9) substituted or unsubstituted aryl,
(10) substituted or unsubstituted heteroaryl, and
(11) substituted or unsubstituted heterocyclyl;
R⁴ is hydrogen or substituted or unsubstituted C₁-C₆ alkyl;
R⁵ is hydrogen, alkyl, alkoxy, or halo;
each of R⁶, R⁷, R⁸, and R⁹ are independently selected from the group consisting of hydrogen, alkyl, alkoxy, halo, substituted or unsubstituted aryl, and substituted or unsubstituted heteroaryl; or
a stereoisomer, tautomer, pharmaceutically acceptable salt, or prodrug thereof, and with the proviso that when R^{a} is amino and R⁶, R⁷, R⁸, and R⁹ are hydrogen, then R⁵ is not hydrogen, alkyl, alkoxy, or halo.

In some embodiments, compounds of formula (IIIa) are provided as the first component: 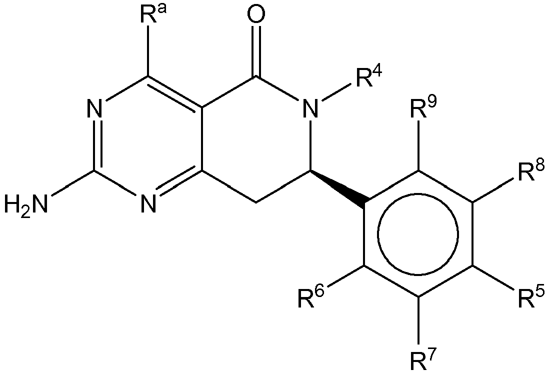 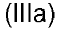 or a tautomer, pharmaceutically acceptable salt, or prodrug thereof, wherein R^{a}, R⁴, R⁵, R⁶, R⁷, R⁸, and R⁹ are as previously defined for formula (III) and with the proviso that when R^{a} is amino and R⁶, R⁷, R⁸, and R⁹ are hydrogen, then R⁵ is not hydrogen, alkyl, alkoxy, or halo.

In some embodiments, R^{a} is hydrogen.

In some embodiments, R^{a} is substituted or unsubstituted C₁-C₆ alkyl.

In some embodiments, R^{a} is C₁-C₆ alkyl or halo C₁-C₆ alkyl. In some such embodiments, R^{a} is methyl.

In some embodiments of the invention, R⁴ is selected from the group consisting of hydrogen, benzyl, 1-(4-methoxyphenyl)ethyl, methyl, 3-aminopropyl, and 2-methyl-2-morpholinopropyl. In other embodiments, R is selected from the group consisting of methyl, ethyl, allyl, 3-methyl-butyl, and isobutyl.

In some embodiments, R⁵ is hydrogen or fluoro. In some aspects, R⁵ is fluoro.

In some embodiments, R⁵ is methyl or methoxy.

In some embodiments, R⁷, R³, and R⁹ are each hydrogen.

In some embodiments, R⁶ is aryl or heteroaryl substituted with one to two substituents selected from the group consisting of halo, alkoxy, alkyl, amino, alkylamino, haloalkyl, and haloalkoxy.

In some embodiments R⁶ is selected from the group consisting of substituted aryl and substituted heteroaryl, wherein said aryl and heteroaryl is selected from the group consisting of furanyl, pyrrolyl, phenyl, pyridyl, pyrazinyl, pyrimidinyl, pyridazinyl, pyrazolyl, imidazolyl, triazolyl, indolyl, oxadiazole, thiadiazole, quinolinyl, isoquinolinyl, isoxazolyl, oxazolyl, thiazolyl, and thienyl. In some aspects, the aforementioned groups are substituted with one to two substituents selected from the group consisting of halo, alkoxy, alkyl, amino, alkylamino, haloalkyl, and haloalkoxy.

In other embodiments R⁶ is selected from the group consisting of (2-hydroxy-ethylamino)-pyrazin-2-yl, 1H-pyrazol-4-yl, 1-methyl-1 H-pyrazol-4-yl, 1-methyl-1 H-pyrazol-4-yl, 2-(5-methyl-pyridin-2-yl)-phenyl, 2,3-difluoro-phenyl, 2,3-dimethoxy-phenyl, 2,4-difluoro-phenyl, 2,4-dimethoxy-phenyl, 2,4-dimethoxy-pyrimidin-5-yl, 2,5-difluoro-phenyl, 2,6-difluoro-phenyl, 2,6-dimethyl-pyridin-3-yl, 2-acetamidophenyl, 2-aminocarbonylphenyl, 2-amino-pyrimidin-5-yl, 2-chloro-4-methoxy-pyrimidin-5-yl, 2-chloro-5-fluoro-pyridin-3-yl, 2-chloro-phenyl, 2-chloro-pyridin-3-yl, 2-chloro-pyridin-4-yl, 2-difluoro-3-methoxyphenyl, 2-ethyl-phenyl, 2-ethoxy-thiazol-4-yl, 2-fluoro-3-methoxy-phenyl, 2-fluoro-3-methylphenyl, 2-fluoro-4-methyl-phenyl, 2-fluoro-5-methoxy-phenyl, 2-fluoro-5-methylphenyl, 2-fluorophenyl, 2-fluoro-pyridin-3-yl, 2-hydroxymethyl-3-methoxyphenyl, 2-hydroxymethylphenyl, 2-isoquinolin-4-yl, 2-methoxy-5-trifluoromethyl-phenyl, 2-methoxy-phenyl, 2-methoxy-pyridin-3-yl, 2-methoxy-pyrimidin-4-yl, 2-methoxy-thiazol-4-yl, 2-methyl-phenyl, 2-methyl-pyridin-3-yl, 2-oxo-1,2-dihydro-pyridin-3-yl, 2-phenoxyphenyl, 2-pyridin-3-yl, 2-pyrimidin-5-yl, 2-trifluoromethoxyphenyl, 2-trifluoromethoxy-phenyl, 3,4-dimethoxy-phenyl, 3,5-dimethyl-isoxazol-4-yl, 3,6-dimethyl-pyrazin-2-yl, 3-acetamidophenyl, 3-aminocarbonylphenyl, 3-bromo-phenyl, 3-chloro-pyrazin-2-yl, 3-cyanophenyl, 3-dimethylaminophenyl, 3-ethoxyphenyl, 3-ethyl-4-methyl-phenyl, 3-ethynyl-phenyl, 3-fluoro-6-methoxy-pyridin-2-yl, 3-fluorophenyl, 3-fluoro-pyrazin-2-yl, 3-methanesulfonamidophenyl, 3-methoxycarbonylphenyl, 3-methoxyphenyl, 3-methoxy-pyrazin-2-yl, 3-methyl-3H-imidazo[4,5-b]pyrazin-5-yl, 3-methylphenyl, 3-methyl-pyridin-2-yl, 3-trifluoromethoxyphenyl, 3-trifluoromethylphenyl, 4,5-dimethoxy-pyrimidin-2-yl, 4-amino-5-fluoro-pyrimidin-2-yl, 4-chloro-2,5-dimethoxy-phenyl, 4-chloro-2-fluoro-phenyl, 4-chloro-2-methoxy-5-methyl-phenyl, 4-chloro-pyridin-3-yl, 4-difluoro-2-methyl-phenyl, 4-ethoxy-5-fluoro-pyrimidin-2-yl, 4-ethoxy-pyrimidin-2-yl, 4-ethoxy-pyrimidin-5-yl, 4-ethyl-1 H-pyrazol-3-yl, 4-fluoro-2-methoxy-phenyl, 4-fluoro-2-methyl-phenyl, 4-fluorophenyl, 4-methoxy-5-methyl-pyrimidin-2-yl, 4-methoxy-pyridin-3-yl, 4-methoxy-pyrimidin-2-yl, 4-methoxy-pyrimidin-5-yl, 4-methyl-phenyl, 4-methyl-pyridin-2-yl, 4-methyl-pyridin-3-yl, 4-pyrrolidin-1-yl-pyrimidin-2-yl, 5,6-dimethoxy-pyrazin-2-yl, 5-acetyl-thiophen-2-yl, 5-amino-6-ethoxy-pyrazin-2-yl, 5-amino-6-methoxy-3-methyl-pyrazin-2-yl, 5-amino-6-methoxy-pyridin-2-yl, 5-chloro-4-methoxy-pyrimidin-2-yl, 5-chloro-6-methoxy-pyrazin-2-yl, 5-dimethylamino-6-methoxy-pyrazin-2-yl, 5-fluoro-2-methoxyphenyl, 5-fluoro-4-methoxy-pyrimidin-2-yl, 5-fluoro-6-methoxy-pyrazin-2-yl, 5-fluoro-pyridin-2-yl, 5-methoxy-pyridin-3-yl, 5-methoxy-thiophen-2-yl, 5-trifluoromethyl-pyrimidin-2-yl, 6-acetyl-pyridin-2-yl, 6-chloro-pyrazin-2-yl, 6-ethoxy-pyrazin-2-yl, 6-ethoxy-pyridin-2-yl, 6-fluoro-pyridin-2-yl, 6-fluoro-pyridin-3-yl, 6-hydroxy-pyridin-2-yl, 6-methoxy-5-methylamino-pyrazin-2-yl, 6-methoxy-5-methyl-pyrazin-2-yl, 6-methoxy-pyrazin-2-yl, 6-methoxy-pyridin-2-yl, 6-methoxy-pyridin-3-yl, 6-methylamino-pyrazin-2-yl, 6-methyl-pyridin-2-yl, 5-amino-6-(2,2,2-trifluoroethoxy)pyrazin-2-yl, and 6-trifluoromethyl-pyridin-2-yl.

The first component and the second component may be provided in a pharmaceutically acceptable carrier to form a pharmaceutical composition.

In another embodiment of the invention, compounds of formula (IV) are provided as the first component: 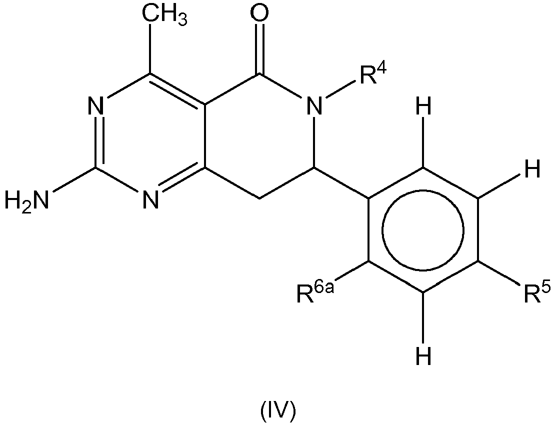 or a stereoisomer, tautomer, pharmaceutically acceptable salt, or prodrug thereof, wherein
R⁴ is hydrogen or substituted or unsubstituted C₁-C₆ alkyl,
R⁵ is hydrogen or halo,
R^{6a} is selected from the group consisting of halo, substituted or unsubstituted aryl, and substituted or unsubstituted heteroaryl.

In some embodiments compounds of formula (IVa) are provided as the first component: 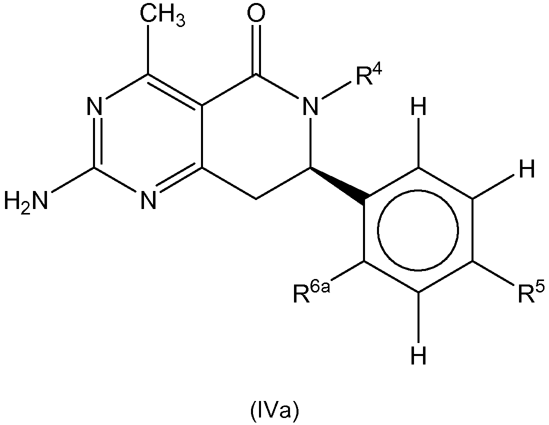 or a tautomer, pharmaceutically acceptable salt, or prodrug thereof, wherein
R⁴, R⁵, and R^{6a} are as previously defined for formula (IV).

In some embodiments of the compounds of formula (IV) or (IVa), R⁴ is selected from the group consisting of hydrogen, benzyl, 1-(4-methoxyphenyl)ethyl, methyl, 3-aminopropyl, and 2-methyl-2-morpholinopropyl. In other embodiments, R is selected from the group consisting of methyl, ethyl, allyl, 3-methyl-butyl, and isobutyl.

In some embodiments, R⁵ is hydrogen or fluoro. In some aspects R⁵ is fluoro.

In some aspects, R^{6a} is aryl or heteroaryl substituted with one to two substituents selected from the group consisting of halo, alkoxy, alkyl, amino, alkylamino, haloalkyl, and haloalkoxy.

In some embodiments R^{6a} is selected from the group consisting of substituted aryl and substituted heteroaryl, wherein said aryl and heteroaryl is selected from the group consisting of furanyl, pyrrolyl, phenyl, pyridyl, pyrazinyl, pyrimidinyl, pyridazinyl, pyrazolyl, imidazolyl, triazolyl, indolyl, oxadiazole, thiadiazole, quinolinyl, isoquinolinyl, isoxazolyl, oxazolyl, thiazolyl, and thienyl. In some aspects, the aforementioned groups are substituted with one to two substituents selected from the group consisting of halo, alkoxy, alkyl, amino, alkylamino, haloalkyl, and haloalkoxy.

In some embodiments, R^{6a} is selected from the group consisting of (2-hydroxy-ethylamino)-pyrazin-2-yl, 1H-pyrazol-4-yl, 1-methyl-1 H-pyrazol-4-yl, 1-methyl-1 H-pyrazol-4-yl, 2-(5-methyl-pyridin-2-yl)-phenyl, 2,3-difluoro-phenyl, 2,3-dimethoxy-phenyl, 2,4-difluoro-phenyl, 2,4-dimethoxy-phenyl, 2,4-dimethoxy-pyrimidin-5-yl, 2,5-difluoro-phenyl, 2,6-difluoro-phenyl, 2,6-dimethyl-pyridin-3-yl, 2-acetamidophenyl, 2-aminocarbonylphenyl, 2-amino-pyrimidin-5-yl, 2-chloro-4-methoxy-pyrimidin-5-yl, 2-chloro-5-fluoro-pyridin-3-yl, 2-chloro-phenyl, 2-chloro-pyridin-3-yl, 2-chloro-pyridin-4-yl, 2-difluoro-3-methoxyphenyl, 2-ethyl-phenyl, 2-ethoxy-thiazol-4-yl, 2-fluoro-3-methoxy-phenyl, 2-fluoro-3-methylphenyl, 2-fluoro-4-methyl-phenyl, 2-fluoro-5-methoxy-phenyl, 2-fluoro-5-methylphenyl, 2-fluorophenyl, 2-fluoro-pyridin-3-yl, 2-hydroxymethyl-3-methoxyphenyl, 2-hydroxymethylphenyl, 2-isoquinolin-4-yl, 2-methoxy-5-trifluoromethyl-phenyl, 2-methoxy-phenyl, 2-methoxy-pyridin-3-yl, 2-methoxy-pyrimidin-4-yl, 2-methoxy-thiazol-4-yl, 2-methyl-phenyl, 2-methyl-pyridin-3-yl, 2-oxo-1,2-dihydro-pyridin-3-yl, 2-phenoxyphenyl, 2-pyridin-3-yl, 2-pyrimidin-5-yl, 2-trifluoromethoxyphenyl, 2-trifluoromethoxy-phenyl, 3,4-dimethoxy-phenyl, 3,5-dimethyl-isoxazol-4-yl, 3,6-dimethyl-pyrazin-2-yl, 3-acetamidophenyl, 3-aminocarbonylphenyl, 3-bromo-phenyl, 3-chloro-pyrazin-2-yl, 3-cyanophenyl, 3-dimethylaminophenyl, 3-ethoxyphenyl, 3-ethyl-4-methyl-phenyl, 3-ethynyl-phenyl, 3-fluoro-6-methoxy-pyridin-2-yl, 3-fluorophenyl, 3-fluoro-pyrazin-2-yl, 3-methanesulfonamidophenyl, 3-methoxycarbonylphenyl, 3-methoxyphenyl, 3-methoxy-pyrazin-2-yl, 3-methyl-3H-imidazo[4,5-b]pyrazin-5-yl, 3-methylphenyl, 3-methyl-pyridin-2-yl, 3-trifluoromethoxyphenyl, 3-trifluoromethylphenyl, 4,5-dimethoxy-pyrimidin-2-yl, 4-amino-5-fluoro-pyrimidin-2-yl, 4-chloro-2,5-dimethoxy-phenyl, 4-chloro-2-fluoro-phenyl, 4-chloro-2-methoxy-5-methyl-phenyl, 4-chloro-pyridin-3-yl, 4-difluoro-2-methyl-phenyl, 4-ethoxy-5-fluoro-pyrimidin-2-yl, 4-ethoxy-pyrimidin-2-yl, 4-ethoxy-pyrimidin-5-yl, 4-ethyl-1 H-pyrazol-3-yl, 4-fluoro-2-methoxy-phenyl, 4-fluoro-2-methyl-phenyl, 4-fluorophenyl, 4-methoxy-5-methyl-pyrimidin-2-yl, 4-methoxy-pyridin-3-yl, 4-methoxy-pyrimidin-2-yl, 4-methoxy-pyrimidin-5-yl, 4-methyl-phenyl, 4-methyl-pyridin-2-yl, 4-methyl-pyridin-3-yl, 4-pyrrolidin-1-yl-pyrimidin-2-yl, 5,6-dimethoxy-pyrazin-2-yl, 5-acetyl-thiophen-2-yl, 5-amino-6-ethoxy-pyrazin-2-yl, 5-amino-6-methoxy-3-methyl-pyrazin-2-yl, 5-amino-6-methoxy-pyridin-2-yl, 5-chloro-4-methoxy-pyrimidin-2-yl, 5-chloro-6-methoxy-pyrazin-2-yl, 5-dimethylamino-6-methoxy-pyrazin-2-yl, 5-fluoro-2-methoxyphenyl, 5-fluoro-4-methoxy-pyrimidin-2-yl, 5-fluoro-6-methoxy-pyrazin-2-yl, 5-fluoro-pyridin-2-yl, 5-methoxy-pyridin-3-yl, 5-methoxy-thiophen-2-yl, 5-trifluoromethyl-pyrimidin-2-yl, 6-acetyl-pyridin-2-yl, 6-chloro-pyrazin-2-yl, 6-ethoxy-pyrazin-2-yl, 6-ethoxy-pyridin-2-yl, 6-fluoro-pyridin-2-yl, 6-fluoro-pyridin-3-yl, 6-hydroxy-pyridin-2-yl, 6-methoxy-5-methylamino-pyrazin-2-yl, 6-methoxy-5-methyl-pyrazin-2-yl, 6-methoxy-pyrazin-2-yl, 6-methoxy-pyridin-2-yl, 6-methoxy-pyridin-3-yl, 6-methylamino-pyrazin-2-yl, 6-methyl-pyridin-2-yl, 5-amino-6-(2,2,2-trifluoroethoxy)pyrazin-2-yl, and 6-trifluoromethyl-pyridin-2-yl.

Preferred Hsp90 inhibitor compounds used as the first component of combination according to the invention include:
(R)-2-amino-7-[2-(2-fluoro-pyridin-3-yl)-phenyl]-4-methyl-7,8-dihydro-6H-pyrido[4,3-d]pyrimidin-5-one;
(S)-2-amino-6-benzyl-7-[4-fluoro-2-(2-fluoro-pyridin-3-yl)-phenyl]-4-methyl-7,8-dihydro-6H-pyrido[4,3-d]pyrimidin-5-one;
(R)-2-amino-7-[4-fluoro-2-(2-fluoro-pyridin-3-yl)-phenyl]-4-methyl-7,8-dihydro-6H-pyrido[4,3-d]pyrimidin-5-one;
(R)-2-amino-7-(2-bromo-4-fluoro-phenyl)-6-[(S)-1-(4-methoxy-phenyl)-ethyl]-4-methyl-7,8-dihydro-6H-pyrido[4,3-d]pyrimidin-5-one;
(R)-2-amino-7-[2-(6-methoxy-pyridin-2-yl)-phenyl]-4-methyl-7,8-dihydro-6H-pyrido[4,3-d]pyrimidin-5-one;
(R)-2-amino-7-[4-fluoro-2-(6-methoxy-pyridin-2-yl)-phenyl]-4-methyl-7,8-dihydro-6H-pyrido[4,3-d]pyrimidin-5-one;
2-amino-7-[4-fluoro-2-(6-methoxy-pyridin-2-yl)-phenyl]-4,6-dimethyl-7,8-dihydro-6H-pyrido[4,3-d]pyrimidin-5-one;
2-amino-7-[4-fluoro-2-(2-fluoro-pyridin-3-yl)-phenyl]-4,6-dimethyl-7,8-dihydro-6H-pyrido[4,3-d]pyrimidin-5-one;
2-amino-7-[4-fluoro-2-(6-methoxypyridin-2-yl)phenyl]-4-methyl-7,8-dihydropyrido[4,3-d]pyrimidin-5(6H)-one;
2-amino-7-[2-(6-methoxy-pyrazin-2-yl)-phenyl]-4-methyl-7,8-dihydro-6H-pyrido[4,3-d]pyrimidin-5-one;
(R)-2-amino-7-[4-fluoro-2-(6-methoxy-pyrazin-2-yl)-phenyl]-4-methyl-7,8-dihydro-6H-pyrido[4,3-d]pyrimidin-5-one;
2-amino-7-[4-fluoro-2-(6-methoxy-pyrazin-2-yl)-phenyl]-4,6-dimethyl-7,8-dihydro-6H-pyrido[4,3-d]pyrimidin-5-one;
2-amino-7-[2-(2-methoxy-pyridin-3-yl)-phenyl]-4-methyl-7,8-dihydro-6H-pyrido[4,3-d]pyrimidin-5-one;
2-amino-7-(5,2'-difluoro-biphenyl-2-yl)-4-methyl-7,8-dihydro-6H-pyrido[4,3-d]pyrimidin-5-one;
2-amino-7-(5-fluoro-2'-trifluoromethoxy-biphenyl-2-yl)-4-methyl-7,8-dihydro-6H-pyrido[4,3-d]pyrimidin-5-one;
2-amino-7-[2-(2-chloro-pyridin-3-yl)-4-fluoro-phenyl]-4-methyl-7,8-dihydro-6H-pyrido[4,3-d]pyrimidin-5-one;
2-amino-7-[4-fluoro-2-(6-fluoro-pyridin-3-yl)-phenyl]-4-methyl-7,8-dihydro-6H-pyrido[4,3-d]pyrimidin-5-one;
2-amino-7-(4-fluoro-2-isoquinolin-4-yl-phenyl)-4-methyl-7,8-dihydro-6H-pyrido[4,3-d]pyrimidin-5-one;
2-amino-7-(5,3'-difluoro-biphenyl-2-yl)-4-methyl-7,8-dihydro-6H-pyrido[4,3-d]pyrimidin-5-one;
2-amino-7-[2-(4-chloro-pyridin-3-yl)-4-fluoro-phenyl]-4-methyl-7,8-dihydro-6H-pyrido[4,3-d]pyrimidin-5-one;
2-amino-7-(5,2'-difluoro-3'-methoxy-biphenyl-2-yl)-4-methyl-7,8-dihydro-6H-pyrido[4,3-d]pyrimidin-5-one;
2-amino-7-(5,4'-difluoro-2'-methyl-biphenyl-2-yl)-4-methyl-7,8-dihydro-6H-pyrido[4,3-d]pyrimidin-5-one;
2-amino-7-(5-fluoro-2'-methoxy-biphenyl-2-yl)-4-methyl-7,8-dihydro-6H-pyrido[4,3-d]pyrimidin-5-one;
2-amino-7-(4-fluoro-2-pyrimidin-5-yl-phenyl)-4-methyl-7,8-dihydro-6H-pyrido[4,3-d]pyrimidin-5-one;
2-amino-7-[4-fluoro-2-(2-methoxy-pyridin-3-yl)-phenyl]-4-methyl-7,8-dihydro-6H-pyrido[4,3-d]pyrimidin-5-one;
2-amino-7-(5-fluoro-3'-methoxy-biphenyl-2-yl)-4-methyl-7,8-dihydro-6H-pyrido[4,3-d]pyrimidin-5-one;
(R)-2-amino-6-(3-amino-propyl)-7-[4-fluoro-2-(6-methoxy-pyridin-2-yl)-phenyl]-4-methyl-7,8-dihydro-6H-pyrido[4,3-d]pyrimidin-5-one;
2-amino-7-(4-fluoro-2-pyridin-3-yl-phenyl)-4-methyl-7,8-dihydro-6H-pyrido[4,3-d]pyrimidin-5-one;
2-amino-7-(5,2'-difluoro-4'-methyl-biphenyl-2-yl)-4-methyl-7,8-dihydro-6H-pyrido[4,3-d]pyrimidin-5-one;
2-amino-7-[4-fluoro-2-(1-methyl-1H-pyrazol-4-yl)-phenyl]-4-methyl-7,8-dihydro-6H-pyrido[4,3-d]pyrimidin-5-one;
2-amino-7-[4-fluoro-2-(1H-pyrazol-4-yl)-phenyl]-4-methyl-7,8-dihydro-6H-pyrido[4,3-d]pyrimidin-5-one;
2-amino-4-methyl-7-(5,2',3'-trifluoro-biphenyl-2-yl)-7,8-dihydro-6H-pyrido[4,3-d]pyrimidin-5-one;
2-amino-7-(2-bromo-4-fluoro-phenyl)-4-methyl-6-(2-methyl-2-morpholin-4-yl-propyl)-7,8-dihydro-6H-pyrido[4,3-d]pyrimidin-5-one;
2-amino-7-(3'-dimethylamino-5-fluoro-biphenyl-2-yl)-4-methyl-7,8-dihydro-6H-pyrido[4,3-d]pyrimidin-5-one;
2-amino-7-[2-(2,4-dimethoxy-pyrimidin-5-yl)-4-fluoro-phenyl]-4-methyl-7,8-dihydro-6H-pyrido[4,3-d]pyrimidin-5-one;
2-amino-7-[4-fluoro-2-(5-methoxy-pyridin-3-yl)-phenyl]-4-methyl-7,8-dihydro-6H-pyrido[4,3-d]pyrimidin-5-one;
2-amino-7-(4-fluoro-2-pyrimidin-5-yl-phenyl)-4-methyl-6-(2-methyl-2-morpholin-4-yl-propyl)-7,8-dihydro-6H-pyrido[4,3-d]pyrimidin-5-one;
2-amino-7-[4-fluoro-2-(2-methoxy-pyridin-3-yl)-phenyl]-4-methyl-6-(2-methyl-2-morpholin-4-yl-propyl)-7,8-dihydro-6H-pyrido[4,3-d]pyrimidin-5-one;
2-amino-7-(5-fluoro-3'-methoxy-biphenyl-2-yl)-4-methyl-6-(2-methyl-2-morpholin-4-yl-propyl)-7,8-dihydro-6H-pyrido[4,3-d]pyrimidin-5-one;
(R)-2-amino-7-[4-fluoro-2-(4-methoxy-5-methyl-pyrimidin-2-yl)-phenyl]-4-methyl-7,8-dihydro-6H-pyrido[4,3-d]pyrimidin-5-one;
2-amino-7-(4-fluoro-2-furan-3-yl-phenyl)-4-methyl-7,8-dihydro-6H-pyrido[4,3-d]pyrimidin-5-one, and
stereoisomers, tautomers, and pharmaceutically acceptable salts or prodrugs thereof.

Examples of the foregoing Hsp90 inhibitor compounds of Formula (I) and methods of making the same are disclosed in U.S. Patent Application Publication No. 2007-0123546 A1, published May 31, 2007, which is incorporated herein by reference in its entirety.

The following definitions are provided to better understand the invention.

"Alkyl" or "unsubstituted alkyl" refers to saturated hydrocarbyl groups that do not contain heteroatoms. Thus the phrase includes straight chain alkyl groups such as methyl, ethyl, propyl, butyl, pentyl, hexyl, heptyl, octyl, nonyl, decyl, undecyl, dodecyl and the like. The phrase also includes branched chain isomers of straight chain alkyl groups, including but not limited to, the following which are provided by way of example: -CH(CH₃)₂, -CH(CH₃)(CH₂CH₃), -CH(CH₂CH₃)₂, -C(CH₃)₃, -C(CH₂CH₃)₃, -CH₂CH(CH₃)₂, -CH₂CH(CH₃)(CH₂CH₃), -CH₂CH(CH₂CH₃)₂, -CH₂C(CH₃)₃, -CH₂C(CH₂CH₃)₃, -CH(CH₃)CH(CH₃)(CH₂CH₃), -CH₂CH₂CH(CH₃)₂, -CH₂CH₂CH(CH₃)(CH₂CH₃), -CH₂CH₂CH(CH₂CH₃)₂, -CH₂CH₂C(CH₃)₃, -CH₂CH₂C(CH₂CH₃)₃, -CH(CH₃)CH₂CH(CH₃)₂, -CH(CH₃)CH(CH₃)CH(CH₃)₂, -CH(CH₂CH₃)CH(CH₃)CH(CH₃)(CH₂CH₃), and others. Thus the phrase "alkyl groups" includes primary alkyl groups, secondary alkyl groups, and tertiary alkyl groups. Preferred alkyl groups include straight and branched chain alkyl groups having 1 to 12, 1 to 6, or 1 to 3 carbon atoms.

"Alkylene" or "unsubstituted alkylene" refers to the same residues as noted above for "alkyl," but having two points of attachment. Exemplary alkylene groups include ethylene (-CH₂CH₂-), propylene (-CH₂CH₂CH₂-), and dimethylpropylene (-CH₂C(CH₃)₂CH₂-). "Alkenyl" or "unsubstituted alkenyl" refers to straight chain and branched, chain hydrocarbyl radicals having one or more carbon-carbon double bonds and from 2 to about 20 carbon atoms. Preferred alkenyl groups include straight chain and branched alkenyl groups having 2 to 12, or 2 to 6 carbon atoms.

"Alkynyl" or "unsubstituted alkynyl" refers to straight chain and branched chain hydrocarbyl radicals having one or more carbon-carbon triple bonds and from 2 to about 20 carbon atoms. Preferred alkynyl groups include straight chain and branched alkynyl groups having 2 to 12, or 2 to 6 carbon atoms.

"Cycloalkyl" or "unsubstituted cycloalkyl" refers to a mono- or polycyclic alkyl substituent. Representative cycloalkyl groups include cyclopropyl, cyclobutyl, cyclopentyl, cyclohexyl, cycloheptyl, and cyclooctyl. Preferred cycloalkyl groups have 3 to 7 carbon atoms.

"Cycloalkenyl" or "unsubstituted cycloalkenyl" refers to a mono- or polycyclic alkyl substituents having at least one ring carbon-carbon double bond. Preferred cycloalkenyl groups have 5 to 7 carbon atoms and include cyclopentenyl and cyclohexenyl. "Substituted alkyl" refers to an alkyl group as defined above in which one or more bonds to a carbon(s) or hydrogen(s) are replaced by a bond to non-hydrogen or non-carbon atoms such as, but not limited to, a halogen atom such as F, Cl, Br, and I; an oxygen atom in groups such as hydroxyl groups, alkoxy groups, aryloxy groups, and ester groups; a sulfur atom in groups such as thiol groups, alkyl and aryl sulfide, sulfone, sulfonyl, and sulfoxide groups; a nitrogen atom in groups such as amino, amido, alkylamino, arylamino, alkylarylamino, diarylamino, N-oxides, imides, and enamines. Substituted alkyl groups also include groups in which one or more bonds to a carbon(s) or hydrogen(s) atom is replaced by a higher-order bond (e.g., a double- or triple-bond) to a heteroatom such as oxygen in oxo, carbonyl, carboxyl, and ester groups; or nitrogen in groups such as imines, oximes, hydrazones, and nitriles. Substituted alkyl groups further include alkyl groups in which one or more bonds to a carbon(s) or hydrogen(s) atoms is replaced by a bond to an aryl, heteroaryl, heterocyclyl, cycloalkyl, or cycloalkenyl group. Preferred substituted alkyl groups include, among others, alkyl groups in which one or more bonds to a carbon or hydrogen atom is/are replaced by one or more bonds to fluoro, chloro, or bromo group. Another preferred substituted alkyl group is the trifluoromethyl group and other alkyl groups that contain the trifluoromethyl group. Other preferred substituted alkyl groups include those in which one or more bonds to a carbon or hydrogen atom is replaced by a bond to an oxygen atom such that the substituted alkyl group contains a hydroxyl, alkoxy, or aryloxy group. Other preferred substituted alkyl groups include alkyl groups that have an amino, or a substituted or unsubstituted alkylamino, arylamino, heterocyclylamino. Still other preferred substituted alkyl groups include those in which one or more bonds to a carbon(s) or hydrogen(s) atoms is replaced by a bond to an aryl, heteroaryl, heterocyclyl, or cycloalkyl group. Examples of substituted alkyl are: -(CH₂)₃NH₂, -(CH₂)₃NH(CH₃), -(CH₂)₃NH(CH₃)₂, -CH₂C(=CH₂)CH₂NH₂,-CH₂C(=O)CH₂NH₂,-CH₂S(=O)₂CH₃,-CH₂OCH₂NH₂, -CH₂CO₂H. Examples of substituents of substituted alkyl are: -CH₂OH, -OH, -OCH₃, -OC₂H₅, -OCF₃, OC(=O)CH₃, -OC(=O)NH₂,-OC(=O)N(CH₃)₂,-CN, -NO₂, -C(=O)CH₃, -CO₂H, -CO₂CH₃, -CONH₂, -NH₂, -N(CH₃)₂, -NHSO₂CH₃, -NHCOCH₃, -NHC(=O)OCH₃, -NHSO-₂CH₃, -SO₂CH₃, -SO₂NH₂, and halo.

"Substituted alkenyl" has the same meaning with respect to unsubstituted alkenyl groups that substituted alkyl groups has with respect to unsubstituted alkyl groups. A substituted alkenyl group includes alkenyl groups in which a non-carbon or non-hydrogen atom is bonded to a carbon double bonded to another carbon and those in which one of the non-carbon or non-hydrogen atoms is bonded to a carbon not involved in a double bond to another carbon.

"Substituted alkynyl" has the same meaning with respect to unsubstituted alkynyl groups that substituted alkyl groups has with respect to unsubstituted alkyl groups. A substituted alkynyl group includes alkynyl groups in which a non-carbon or non-hydrogen atom is bonded to a carbon triple bonded to another carbon and those in which a non-carbon or non-hydrogen atom is bonded to a carbon not involved in a triple bond to another carbon. "Substituted cycloalkyl" has the same meaning with respect to unsubstituted cycloalkyl groups that substituted alkyl groups has with respect to unsubstituted alkyl groups. "Substituted cycloalkenyl" has the same meaning with respect to unsubstituted cycloalkenyl groups that substituted alkyl groups has with respect to unsubstituted alkyl groups. "Aryl" or "unsubstituted aryl" refers to monocyclic and polycyclic aromatic groups that do not contain ring heteroatoms. Such groups can contain from 6 to 14 carbon atoms but preferably 6. Exemplary aryl moieties employed as substituents in compounds of the present invention include phenyl, naphthyl, and the like.

"Aralkyl" or "arylalkyl" refers to an alkyl group substituted with an aryl group as defined above. Typically, aralkyl groups employed in compounds of the present invention have from 1 to 6 carbon atoms incorporated within the alkyl portion of the aralkyl group. Suitable aralkyl groups employed in compounds of the present invention include, for example, benzyl and the like. "Heteroarylalkyl" or "heteroaralkyl" refers to an alkyl group substituted with a heteroaryl group as defined above. Typically, heteroarylalkyl groups employed in compounds of the present invention have from 1 to 6 carbon atoms incorporated within the alkyl portion of the aralkyl group. Suitable heteroarylalkyl groups employed in compounds of the present invention include, for example, picolyl and the like.

"Alkoxy" refers to R²⁰O- wherein R²⁰ is C₁-C₇ alkyl or substituted alkyl. In some embodiments, R²⁰ is C₁-C₆ alkyl. Representative examples of alkoxy groups include methoxy, ethoxy, t-butoxy, trifluoromethoxy, and the like.

"Amino" refers herein to the group -NH₂.

"Substituted amino" refers to the group -NR⁶⁰R⁶¹ where R⁶⁰ and R⁶¹ are independently selected from the group consisting of hydrogen, alkyl, substituted alkyl, alkenyl, substituted alkenyl, alkynyl, substituted alkynyl, aryl, substituted aryl, cycloalkyl, substituted cycloalkyl, heteroaryl, substituted heteroaryl, heterocyclic, substituted heterocyclic, -SO₂-alkyl, -SO₂-substituted alkyl, and where R⁶⁰ and R⁶¹ are joined, together with the nitrogen bound thereto to form a heterocyclic or substituted heterocyclic group provided that R⁶⁰ and R⁶¹ are both not hydrogen. When R⁶⁰ is hydrogen and R⁶¹ is alkyl, the substituted amino group is sometimes referred to herein as alkylamino. When R⁶⁰ and R⁶¹ are alkyl, the substituted amino group is sometimes referred to herein as dialkylamino. When referring to a monosubstituted amino, it is meant that either R⁶⁰ and R⁶¹ is hydrogen but not both. When referring to a disubstituted amino, it is meant that neither R⁶⁰ and R⁶¹ is hydrogen. The term "alkylamino" refers herein to the group -NR⁶⁰R⁶¹ where R⁶⁰ is C₁-C₇ alkyl and R⁶⁰ is hydrogen or C₁-C₇ alkyl. The term "dialkylamino" refers to the group -NR⁶⁰R⁶¹ where R⁶⁰ and R⁶¹ are C₁-C₇ alkyl. The term "arylamino" refers herein to the group -NR⁶⁰R⁶¹ where R⁶⁰ is C₅-C₇ aryl and R⁶¹ is hydrogen, C₁-C₇ alkyl, or C₅-C₇ aryl. The term "aralkylamino" refers herein to the group -NR⁶⁰R⁶¹ where R⁶⁰ is aralkyl and R⁶¹ is hydrogen, C₁-C₇ alkyl, C₅-C₇ aryl, or C₅-C₇ aralkyl.

"Amidino" refers to the moieties R⁴⁰-C(=N)-NR⁴¹- (the radical being at the "N¹" nitrogen) and R⁴⁰(NR⁴¹)C=N- (the radical being at the "N²" nitrogen), where R⁴⁰ and R⁴¹ can be hydrogen, C₁-C₇ alkyl, aryl, or C₅-C₇ aralkyl.

"Alkoxyalkyl" refers to the group -alk₁-O-alk₂ where alk₁ is C₁-C₇ alkyl, and alk₂ is C₁-C₇ alkyl. The term "aryloxyalkyl" refers to the group -(C₁-C₇ alkyl)-O-(C₅-C₇ aryl). "Alkoxyalkylamino" refers herein to the group -NR²⁷-(alkoxyalkyl), where R²⁷ is typically hydrogen, C₅-C₇ aralkyl, or C₁-C₇ alkyl.

"Aminocarbonyl" refers herein to the group -C(O)-NH₂. "Substituted aminocarbonyl" refers herein to the group -C(O)-NR²⁸R²⁹ where R²⁸ is C₁-C₇ alkyl and R²⁹ is hydrogen or C₁-C₇ alkyl. The term "arylaminocarbonyl" refers herein to the group -C(O)-NR³⁰R³¹ where R³⁰ is C₅-C₇ aryl and R³¹ is hydrogen, C₁-C₇ alkyl or C₅-C₇ aryl. "Aralkylaminocarbonyl" refers herein to the group -C(O)-NR³²R³³ where R³² is C₅-C₇ aralkyl and R³³ is hydrogen, C₁-C₇ alkyl, C₅-C₇ aryl, or C₅-C₇ aralkyl.

"Aminosulfonyl" refers herein to the group -S(O)₂-NH₂. "Substituted aminosulfonyl" refers herein to the group -S(O)₂-NR³⁴R³⁵ where R³⁴ is C₁-C₇ alkyl and R³⁵ is hydrogen or C₁-C₇ alkyl. The term "aralkylaminosulfonlyaryl" refers herein to the group -(C₅-C₇ aryl)-S(O)₂-NH-aralkyl.

"Aryloxy" refers to R⁵⁰O- wherein R⁵⁰ is aryl.

"Carbonyl" refers to the divalent group -C(O)-. "Alkylcarbonyl' refers to the group

-C(O)alkyl. "Arylcarbonyl" refers to the group -C(O)aryl. Similarly, the term "heteroarylcarbonyl", "aralkylcarbonyl", and "heteroaralkylcarbonyl" refers to -C(O)-R where R is respectively heteroaryl, aralkyl, and heteroaralkyl.

"Carbonyloxy" refers generally to the group -C(O)-O-. Such groups include esters, -C(O)-O-R³⁶, where R³⁶ is C₁-C₇ alkyl, C₃-C₇ cycloalkyl, aryl, or C₅-C₇ aralkyl. The term "arylcarbonyloxy" refers herein to the group -C(O)-O-(aryl). The term "aralkylcarbonyloxy" refers herein to the group -C(O)-O-(C₅-C₇ aralkyl).

"Cycloalkylalkyl" refers to an alkyl group substituted with a cyloalkyl group as defined above. Typically, cycloalkylalkyl groups have from 1 to 6 carbon atoms incorporated within the alkyl portion of the cycloalkylalkyl group.

"Carbonylamino" refers to the divalent group -NH-C(O)- in which the hydrogen atom of the amide nitrogen of the carbonylamino group can be replaced C₁-C₇ alkyl, aryl, or C₅-C₇ aralkyl group. Carbonylamino groups include moieties such as carbamate esters (-NH-C(O)-O-R²⁸) and amido -NH-C(O)-R²⁸, where R²⁸ is a straight or branched chain C₁-C₇ alkyl, C₃-C₇ cycloalkyl, or aryl or C₅-C₇ aralkyl. The term "alkylcarbonylamino" refers to the group -NH-C(O)-R^{28'} where R^{28'} is alkyl having from 1 to about 7 carbon atoms in its backbone structure. The term "arylcarbonylamino" refers to group -NH-C(O)-R²⁹ where R²⁹ is C₅-C₇ aryl. Similarly, the term "aralkylcarbonylamino" refers to carbonylamino where R²⁹ is C₅-C₇ aralkyl.

"Guanidino" or "guanidyl" refers to moieties derived from guanidine, H₂N-C(=NH)-NH₂. Such moieties include those bonded at the nitrogen atom carrying the formal double bond (the "2"-position of the guanidine, e.g., diaminomethyleneamino, (H₂N)₂C=NH-) and those bonded at either of the nitrogen atoms carrying a formal single bond (the "1-" and/or "3"-positions of the guandine, e.g., H₂N-C(=NH)-NH-). The hydrogen atoms at any of the nitrogens can be replaced with a suitable substituent, such as C₁-C₇ alkyl, aryl, or C₅-C₇ aralkyl.

"Halogen" or "halo" refers to chloro, bromo, fluoro, and iodo groups. The term "haloalkyl" refers to an alkyl radical substituted with one or more halogen atoms. "Haloalkyl" groups include -CF₃. The term "haloalkoxy" refers to an alkoxy radical substituted with one or more halogen atoms. "Haloalkoxy" groups include -OCF₃ and -OCH₂CF₃.

"Hydroxyl" or "hydroxy" refers to the group -OH.

"Heterocyclic" or "unsubstituted heterocyclic group," "heterocycle" or "unsubstituted heterocycle," and "heterocyclyl" or "unsubstituted heterocyclyl," "heterocycloalkyl" or "unsubstituted heterocycloalkyl group," as used herein refers to any non-aromatic monocyclic or polycyclic ring compounds containing a heteroatom selected from nitrogen, oxygen, or sulfur. Examples include 3- or 4-membered ring containing a heteroatom selected from nitrogen, oxygen, and sulfur or a 5- or 6-membered ring containing from one to three heteroatoms selected from the group consisting of nitrogen, oxygen, or sulfur; wherein the 5-membered ring has 0-1 double bonds and the 6-membered ring has 0-2 double bonds; wherein the nitrogen and sulfur atom maybe optionally oxidized; wherein the nitrogen and sulfur heteroatoms maybe optionally quarternized; and including any bicyclic group in which any of the above heterocyclic rings is fused to a benzene ring or another 5-or 6-membered heterocyclic ring independently defined above provided that the point of attachment is through the heterocyclic ring.

Heterocyclic moieties can be, for example monosubstituted or disubstituted with various substituents independently selected from but not limited to hydroxy, alkoxy, halo, oxo (C=O), alkylimino (R³¹N=, wherein R³¹ is alkyl or alkoxy group), amino, alkylamino, acylaminoalkyl, alkoxy, thioalkoxy, polyalkoxy, alkyl, cycloalkyl or haloalkyl. The heterocyclic groups may be attached at various positions as shown below as will be apparent to those having skill in the organic and medicinal chemistry arts in conjunction with the disclosure herein. 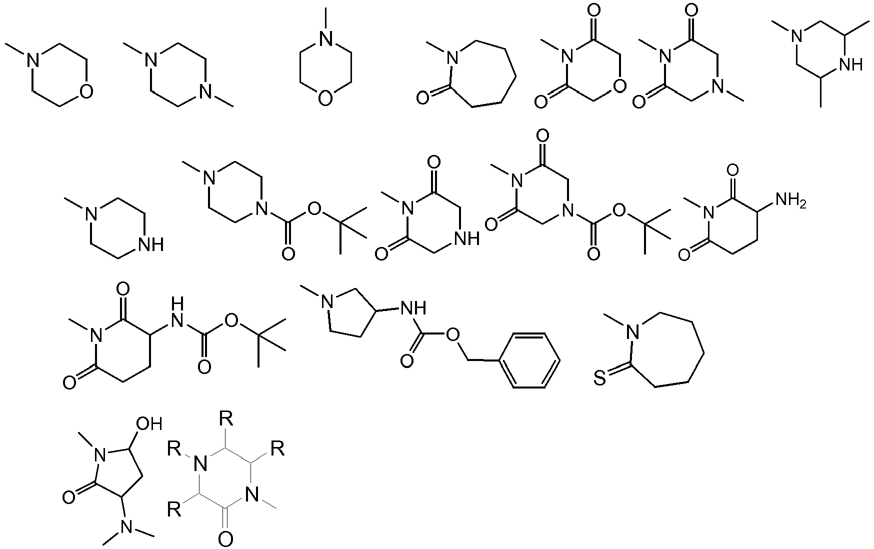 where R is H or a heterocyclic substituent, as described herein.

"Heteroaryl" or "unsubstituted heteroaryl" refers herein to an aromatic group having from 1 to 4 heteroatoms as ring atoms in an aromatic ring with the remainder of the ring atoms being carbon atoms. The term "heteroaryl" includes rings in which nitrogen is the heteroatom as well as partially and fully-saturated rings in which at least one cyclic structure is aromatic, such as, for example, benzodioxozolo (which has a heterocyclic structure fused to a phenyl group, i.e., 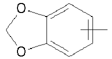 provided that the point of attachment is through the heteroaryl ring. Heteroaryl groups can be further substituted and may be attached at various positions as will be apparent to those having skill in the organic and medicinal chemistry arts in conjunction with the disclosure herein. Representative substituted and unsubstituted heteroaryl groups include, for example, those found in the compounds disclosed in this application and in the examples shown below

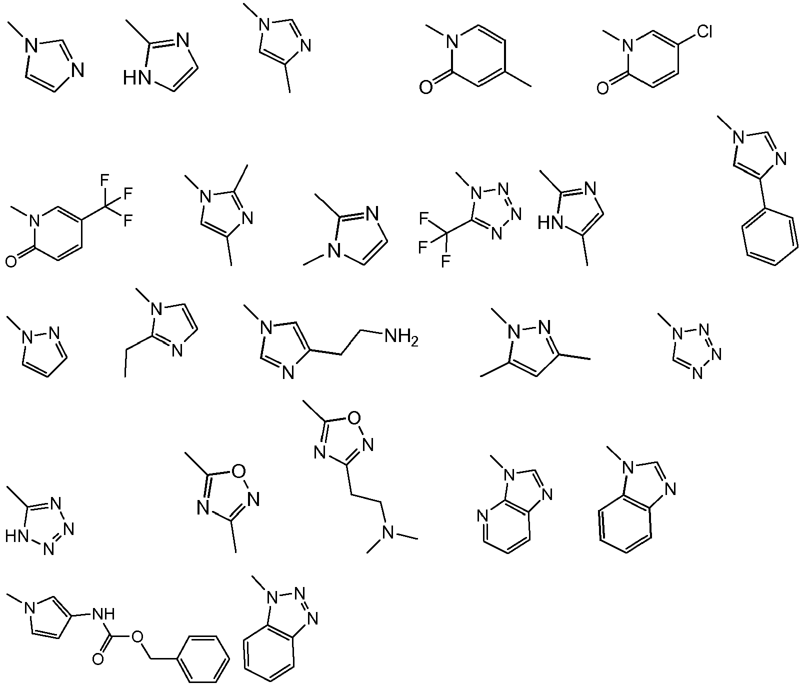

Preferred heterocycles and heteroaryls have 3 to 14 ring atoms and include, for example: diazapinyl, pyrroyl, pyrrolidinyl, pyrazolyl, pyrazolidinyl, imidazoyl, imidazolidinyl, pyridyl, piperidinyl, pyrazinyl, piperazinyl, azetidinyl, pyrimidinyl, pyridazinyl, oxazolyl, oxazolidinyl, isoxazolyl, isoxazolidinyl, morpholinyl, thiazolyl, thiazolidinyl, isothiazolyl, isothiazolidinyl, indolyl, quinolinyl, isoquinolinyl, benzimidazolyl, benzothiazolyl, benzoxazolyl, furyl, thienyl, triazolyl, quinoxalinyl, phthalazinyl, naphthpyridinyl, indazolyl, and benzothienyl. "Heteroarylalkyl" or "heteroaralkyl" refers to an alkyl group substituted with a heteroaryl group as defined above. Typically, heteroarylalkyl groups have from 1 to 6 carbon atoms incorporated within the alkyl portion of the heteroarylalkyl group.

"Imino" refers to the group =NH.

"Nitro" refers to the group NO₂.

"Sulfonyl" refers herein to the group -SO₂-. "Alkylsulfonyl" refers to a substituted sulfonyl of the structure -SO₂R⁵²- in which R⁵² is C₁-C₇ alkyl. Alkylsulfonyl groups employed in compounds of the present invention are typically alkylsulfonyl groups having from 1 to 6 carbon atoms in its backbone structure. Thus, typical alkylsulfonyl groups employed in compounds of the present invention include, for example, methylsulfonyl (i.e., where R⁵² is methyl), ethylsulfonyl (i.e., where R⁵² is ethyl), propylsulfonyl (i.e., where R⁵² is propyl), and the like. The term "arylsulfonyl" refers herein to the group -SO₂-aryl. The term "heterocyclylsulfonyl" refers herein to the group -SO₂-heterocyclyl. The term "aralkylsulfonyl" refers herein to the group -SO₂-aralkyl. The term "sulfonamido" refers herein to -SO₂NH₂. The term "sulfonamidoalkyl" refers to (alkyl)SO₂NH₂-. "Thio" or "thiol" refers to the group -SH. "Alkylthio" or "alkylthiol" refers to a thio group substituted with an alkyl group such as, for example, a C₁-C₆ alkyl group. "Thioamido" refers to the group -C(=S)NH₂.

"Optionally substituted" refers to the optional replacement of hydrogen with a monovalent or divalent radical. "Substituted" refers to the replacement of hydrogen with a monovalent or divalent radical. Unless indicated otherwise, suitable substitution groups include, for example, hydroxyl, alkoxy, nitro, amino, imino, cyano, halo, thio, sulfonyl, thioamido, amidino, oxo, oxamidino, methoxamidino, guanidino, sulfonamido, carboxyl, formyl, alkyl, haloalkyl, alkylamino, haloalkylamino, alkoxy, haloalkoxy, alkoxy-alkyl, alkylcarbonyl, aminocarbonyl, arylcarbonyl, aralkylcarbonyl, heteroarylcarbonyl, heteroaralkyl-carbonyl, alkylthio, aminoalkyl, cyanoalkyl, aryl and the like. Other suitable substitution groups include those substituents indicated for substituted alkyl. Examples of various suitable substitution groups are also found in reference to the compounds disclosed throughout this application.

The substitution group can itself be substituted. The group substituted onto the substitution group can be carboxyl, halo, nitro, amino, cyano, hydroxyl, alkyl, alkoxy, aminocarbonyl, - SR⁴², thioamido, -SO₃H, -SO₂R⁴², or cycloalkyl, where R⁴² is typically hydrogen, hydroxyl or alkyl.

When the substituted substituent includes a straight chain group, the substitution can occur either within the chain (e.g., 2-hydroxypropyl, 2-aminobutyl, and the like) or at the chain terminus (e.g., 2-hydroxyethyl, 3-cyanopropyl, and the like). Substituted substituents can be straight chain, branched or cyclic arrangements of covalently bonded carbon or heteroatoms.

Unless indicated otherwise, the nomenclature of substituents that are not explicitly defined herein are arrived at by naming the terminal portion of the functionality followed by the adjacent functionality toward the point of attachment. For example, the substituent "alkoxyheteroaryl" refers to the group (alkoxy)-(heteroaryl)-.

Preferred compounds of Formula (I) used in this invention have a total molecular weight less than 1000 Daltons, preferably less than 750 Daltons. Compounds of Formula (I) typically have a minimum molecular weight of at least 150 Daltons. Preferred compounds of Formula (I) have a molecular weight between 150 and 750 Daltons, and in more preferred embodiments, have a molecular weight between 200 and 500 Daltons. Other embodiments of the invention include the use of compounds of Formula (I) with a molecular weight between 300 and 450 Daltons. In another aspect of the invention compounds of Formula (I) used in the invention have a molecular weight between 350 and 400 Daltons. Similarly, it is understood that the above definitions are not intended to include impermissible substitution patterns (e.g., methyl substituted with 5 fluoro groups). Such impermissible substitution patterns are well known to the skilled artisan.

"Carboxy-protecting group" refers to a carbonyl group which has been esterified with one of the commonly used carboxylic acid protecting ester groups employed to block or protect the carboxylic acid function while reactions involving other functional sites of the compound are carried out. In addition, a carboxy protecting group can be attached to a solid support whereby the compound remains connected to the solid support as the carboxylate until cleaved by hydrolytic methods to release the corresponding free acid. Representative carboxy-protecting groups include, for example, alkyl esters, secondary amides and the like. Certain of the compounds according to Formula (I) comprise asymmetrically substituted carbon atoms. Such asymmetrically substituted carbon atoms can result in the compounds of the invention comprising mixtures of stereoisomers at a particular asymmetrically substituted carbon atom or a single stereoisomer. As a result, racemic mixtures, mixtures of enantiomers, as well as enantiomers of the compounds of the invention are included in the present invention. The terms "S" and "R" configuration, as used herein, are as defined by the IUPAC 1974 "RECOMMENDATIONS FOR SECTION E, FUNDAMENTAL STEREOCHEMISTRY," Pure Appl. Chem. 45:13-30, 1976. The terms α and β are employed for ring positions of cyclic compounds. The α-side of the reference plane is that side on which the preferred substituent lies at the lower numbered position. Those substituents lying on the opposite side of the reference plane are assigned β descriptor. It should be noted that this usage differs from that for cyclic stereoparents, in which "α" means "below the plane" and denotes absolute configuration. The terms α and β configuration, as used herein, are as defined by the "Chemical Abstracts Index Guide," Appendix IV, paragraph 203, 1987. As used herein, the term "pharmaceutically acceptable salts" refers to the nontoxic acid or alkaline earth metal salts of the 2-amino-7,8-dihydro-6H-pyrido[4,3-d]pyrimidin-5-one compounds of the invention. These salts can be prepared *in situ* during the final isolation and purification of the 2-amino-7,8-dihydro-6H-pyrido[4,3-d]pyrimidin-5-one compounds, or by separately reacting the base or acid functions with a suitable organic or inorganic acid or base, respectively. Representative salts include, but are not limited to, the following: acetate, adipate, alginate, citrate, aspartate, benzoate, benzenesulfonate, bisulfate, butyrate, camphorate, camphorsulfonate, digluconate, cyclopentanepropionate, dodecylsulfate, ethanesulfonate, glucoheptanoate, glycerophosphate, hemi-sulfate, heptanoate, hexanoate, fumarate, hydrochloride, hydrobromide, hydroiodide, 2-hydroxyethanesulfonate, lactate, maleate, methanesulfonate, nicotinate, 2-napthalenesulfonate, oxalate, pamoate, pectinate, persulfate, 3-phenylproionate, picrate, pivalate, propionate, succinate, sulfate, tartrate, thiocyanate, p-toluenesulfonate, and undecanoate. Also, the basic nitrogen-containing groups can be quaternized with such agents as alkyl halides, such as methyl, ethyl, propyl, and butyl chloride, bromides, and iodides; dialkyl sulfates like dimethyl, diethyl, dibutyl, and diamyl sulfates, long chain halides such as decyl, lauryl, myristyl, and stearyl chlorides, bromides and iodides, aralkyl halides like benzyl and phenethyl bromides, and others. Water or oil-soluble or dispersible products are thereby obtained.

Examples of acids that may be employed to form pharmaceutically acceptable acid addition salts include such inorganic acids as hydrochloric acid, sulfuric acid and phosphoric acid and such organic acids as oxalic acid, maleic acid, methanesulfonic acid, succinic acid and citric acid. Basic addition salts can be prepared *in situ* during the final isolation and purification of the 2-amino-7,8-dihydro-6H-pyrido[4,3-d]pyrimidin-5-one compounds, or separately by reacting carboxylic acid moieties with a suitable base such as the hydroxide, carbonate or bicarbonate of a pharmaceutically acceptable metal cation or with ammonia, or an organic primary, secondary or tertiary amine. Pharmaceutically acceptable salts include, but are not limited to, cations based on the alkali and alkaline earth metals, such as sodium, lithium, potassium, calcium, magnesium, aluminum salts and the like, as well as nontoxic ammonium, quaternary ammonium, and amine cations, including, but not limited to ammonium, tetramethylammonium, tetraethylammonium, methylamine, dimethylamine, trimethylamine, triethylamine, ethylamine, and the like. Other representative organic amines useful for the formation of base addition salts include diethylamine, ethylenediamine, ethanolamine, diethanolamine, piperazine, and the like. The term "pharmaceutically acceptable prodrugs" as used herein refers to those prodrugs of the compounds of the present invention which are, within the scope of sound medical judgment, suitable for use in contact with the tissues of humans and lower animals without undue toxicity, irritation, allergic response, and the like, commensurate with a reasonable benefit/risk ratio, and effective for their intended use, as well as the zwitterionic forms, where possible, of the compounds of the invention. The term "prodrug" refers to compounds that are rapidly transformed *in vivo* to yield the parent compound of the above formula, for example by hydrolysis in blood. A thorough discussion is provided in Higuchi, T., and V. Stella, "Pro-drugs as Novel Delivery Systems," A.C.S. Symposium Series 14, and in "Bioreversible Carriers in Drug Design," in Edward B. Roche (ed.), American Pharmaceutical Association, Pergamon Press, 1987, both of which are incorporated herein by reference.

Surprisingly it has now been found that a Hsp90 inhibitor is useful in the treatment of cancer, e.g. solid tumors, e.g. sarcomas, e.g. carcinomas of the bladder, the colon, the rectum, the liver, the lung, e.g. pleural mesothelioma, e.g. non small cell, e.g. small cell, the breast, the vagina, the ovaries, the uterus, the pancreas, the kidney, the stomach, gastrointestinal tract, e.g. gastrointestinal stromal tumor, e.g. the small intestine, e.g. the esophagus, e.g. the bile duct, the prostate, the testis, the head and neck, the peritoneal cavity, the thyroid, the bone, the brain, the central nervous system e.g. glioblastoma, e.g. neuroblastoma, neurofibromatosis, chordoma, thymoma, adenoid cystic carcinoma, aggressive fibromatosis, myelofibrosis, desmoplastic small cell round tumor and/or the skin, e.g. melanoma and/or the endocrine system, e.g. the thyroid, the adrenal gland,and/or cancer of the blood , e.g. hematological cancer, e.g. leukemia, e.g. acute myeloid leukemia, e.g. chronic myeloid leukemia, e.g. chronic lymphatic leukemia, e.g. acute lymphatic leukemia, e.g. multiple myeloma e.g. lymphomas, and/or in treating of myelodysplastic syndrome, systemic mastocytosis, von Hippel-Lindau syndrome, multicentric Castleman disease and/or psioriasis.

Accordingly the present invention provides the use of a Hsp90 for the manufacture of pharmaceutical compositions for use in the treatment of cancer, e.g. solid tumors, e.g. sarcomas, e.g. carcinomas of the bladder, the colon, the rectum, the liver, the lung, e.g. pleural mesothelioma, e.g. non small cell, e.g. small cell, the breast, the vagina, the ovaries, the uterus, the pancreas, the kidney, the stomach, gastrointestinal tract, e.g. gastrointestinal stromal tumor, e.g. the small intestine, e.g. the esophagus, e.g. the bile duct, the prostate, the testis, the head and neck, the peritoneal cavity, the thyroid, the bone, the brain, the central nervous system e.g. glioblastoma, e.g. neuroblastoma, neurofibromatosis, chordoma, thymoma, adenoid cystic carcinoma, aggressive fibromatosis, myelofibrosis, desmoplastic small cell round tumor and/or the skin, e.g. melanoma and/or the endocrine system, e.g. the thyroid, the adrenal gland,and/or cancer of the blood , e.g. hematological cancer, e.g. leukemia, e.g. acute myeloid leukemia, e.g. chronic myeloid leukemia, e.g. chronic lymphatic leukemia, e.g. acute lymphatic leukemia, e.g. multiple myeloma e.g. lymphomas, and/or for use in treatment of myelodysplastic syndrome, systemic mastocytosis, von Hippel-Lindau syndrome, multicentric Castleman disease and/or psioriasis.

In another aspect the present invention provides the use of a Hsp90 inhibitor in the treatment of cancer, e.g. solid tumors, e.g. sarcomas, e.g. carcinomas of the bladder, the colon, the rectum, the liver, the lung, e.g. pleural mesothelioma, e.g. non small cell, e.g. small cell, the breast, the vagina, the ovaries, the uterus, the pancreas, the kidney, the stomach, gastrointestinal tract, e.g. gastrointestinal stromal tumor, e.g. the small intestine, e.g. the esophagus, e.g. the bile duct, the prostate, the testis, the head and neck, the peritoneal cavity, the thyroid, the bone, the brain, the central nervous system e.g. glioblastoma, e.g. neuroblastoma, neurofibromatosis, chordoma, thymoma, adenoid cystic carcinoma, aggressive fibromatosis, myelofibrosis, desmoplastic small cell round tumor and/or the skin, e.g. melanoma and/or the endocrine system, e.g. the thyroid, the adrenal gland,and/or cancer of the blood , e.g. hematological cancer, e.g. leukemia, e.g. acute myeloid leukemia, e.g. chronic myeloid leukemia, e.g. chronic lymphatic leukemia, e.g. acute lymphatic leukemia, e.g. multiple myeloma e.g. lymphomas, and/or in the treatment of myelodysplastic syndrome, systemic mastocytosis, von Hippel-Lindau syndrome, multicentric Castleman disease and/or psioriasis.

In a further aspect the present invention provides a Hsp90 inhibitor in treating cancer, e.g. solid tumors, e.g. sarcomas, e.g. carcinomas of the bladder, the colon, the rectum, the liver, the lung, e.g. pleural mesothelioma, e.g. non small cell, e.g. small cell, the breast, the vagina, the ovaries, the uterus, the pancreas, the kidney, the stomach, gastrointestinal tract, e.g. gastrointestinal stromal tumor, e.g. the small intestine, e.g. the esophagus, e.g. the bile duct, the prostate, the testis, the head and neck, the peritoneal cavity, the thyroid, the bone, the brain, the central nervous system e.g. glioblastoma, e.g. neuroblastoma, neurofibromatosis, chordoma, thymoma, adenoid cystic carcinoma, aggressive fibromatosis, myelofibrosis, desmoplastic small cell round tumor and/or the skin, e.g. melanoma and/or the endocrine system, e.g. the thyroid, the adrenal gland,and/or cancer of the blood , e.g. hematological cancer, e.g. leukemia, e.g. acute myeloid leukemia, e.g. chronic myeloid leukemia, e.g. chronic lymphatic leukemia, e.g. acute lymphatic leukemia, e.g. multiple myeloma e.g. lymphomas, and/or for use in treating myelodysplastic syndrome, systemic mastocytosis, von Hippel-Lindau syndrome, multicentric Castleman disease and/or psioriasis.

In a further aspect the present invention provides a method of treating humans suffering from cancer, e.g. solid tumors, e.g. sarcomas, e.g. carcinomas of the bladder, the colon the rectum, the liver, the lung, e.g. pleural mesothelioma, e.g. non small cell, e.g. small cell, the breast, the vagina, the ovaries, the uterus, the pancreas, the kidney, the stomach, gastrointestinal tract, e.g. gastrointestinal stromal tumor, e.g. the small intestine, e.g. the esophagus, e.g. the bile duct, the prostate, the testis, the head and neck, the peritoneal cavity, the thyroid, the bone, the brain, the central nervous system e.g. glioblastoma, e.g. neuroblastoma, neurofibromatosis, chordoma, thymoma, adenoid cystic carcinoma, aggressive fibromatosis, myelofibrosis, desmoplastic small cell round tumor and/or the skin, e.g. melanoma and/or the endocrine system, e.g. the thyroid, the adrenal gland, and/or cancer of the blood , e.g. hematological cancer, e.g. leukemia, e.g. acute myeloid leukemia, e.g. chronic myeloid leukemia, e.g. chronic lymphatic leukemia, e.g. acute lymphatic leukemia, e.g. multiple myeloma e.g. lymphomas, and/or myelodysplastic syndrome, systemic mastocytosis, von Hippel-Lindau syndrome, multicentric Castleman disease and/or psioriasis which comprises administering to said human in need of such treatment a dose of a Hsp90 inhibitor effective against cancer, e.g. solid tumors, e.g. sarcomas, e.g. carcinomas of the bladder, the colon, the rectum, the liver, the lung, e.g. pleural mesothelioma, e.g. non small cell, e.g. small cell, the breast, the vagina, the ovaries, the uterus, the pancreas, the kidney, the stomach, gastrointestinal tract, e.g. gastrointestinal stromal tumor, e.g. the small intestine, e.g. the esophagus, e.g. the bile duct, the prostate, the testis, the head and neck, the peritoneal cavity, the thyroid, the bone, the brain, the central nervous system e.g. glioblastoma, e.g. neuroblastoma, neurofibromatosis, chordoma, thymoma, adenoid cystic carcinoma, aggressive fibromatosis, myelofibrosis, desmoplastic small cell round tumor and/or the skin, e.g. melanoma and/or the endocrine system, e.g. the thyroid, the adrenal gland,and/or cancer of the blood , e.g. hematological cancer, e.g. leukemia, e.g. acute myeloid leukemia, e.g. chronic myeloid leukemia, e.g. chronic lymphatic leukemia, e.g. acute lymphatic leukemia, e.g. multiple myeloma e.g. lymphomas, and/or myelodysplastic syndrome, systemic mastocytosis, von Hippel-Lindau syndrome, multicentric Castleman disease and/or psioriasis.

In a further aspect the present invention provides a pharmaceutical preparation for the treatment of cancer, e.g. solid tumors, e.g. sarcomas, e.g. carcinomas of the bladder, the colon, the rectum, the liver, the lung, e.g. pleural mesothelioma, e.g. non small cell, e.g. small cell, the breast, the vagina, the ovaries, the uterus, the pancreas, the kidney, the stomach, gastrointestinal tract, e.g. gastrointestinal stromal tumor, e.g. the small intestine, e.g. the esophagus, e.g. the bile duct, the prostate, the testis, the head and neck, the peritoneal cavity, the thyroid, the bone, the brain, the central nervous system e.g. glioblastoma, e.g. neuroblastoma, neurofibromatosis, chordoma, thymoma, adenoid cystic carcinoma, aggressive fibromatosis, myelofibrosis, desmoplastic small cell round tumor and/or the skin, e.g. melanoma and/or the endocrine system, e.g. the thyroid, the adrenal gland,and/or cancer of the blood , e.g. hematological cancer, e.g. leukemia, e.g. acute myeloid leukemia, e.g. chronic myeloid leukemia, e.g. chronic lymphatic leukemia, e.g. acute lymphatic leukemia, e.g. multiple myeloma e.g. lymphomas, and/or myelodysplastic syndrome, systemic mastocytosis, von Hippel-Lindau syndrome, multicentric Castleman disease and/or psioriasis comprising a Hsp90 inhibitor and at least one pharmaceutically acceptable carrier.

Depending on species, age, individual condition, mode of administration, and the clinical picture in question, effective doses for example weekly doses of about 2 to 300 mg, preferably 50 to 160 mg of a Hsp90 inhibitor are administered to a human.

The present invention further provides a method for administering to a human having cancer, e.g. solid tumors, e.g. sarcomas, e.g. carcinomas of the bladder, the colon, the rectum, the liver, the lung, e.g. pleural mesothelioma, e.g. non small cell, e.g. small cell, the breast, the vagina, the ovaries, the uterus, the pancreas, the kidney, the stomach, gastrointestinal tract, e.g. gastrointestinal stromal tumor, e.g. the small intestine, e.g. the esophagus, e.g. the bile duct, the prostate, the testis, the head and neck, the peritoneal cavity, the thyroid, the bone, the brain, the central nervous system e.g. glioblastoma, e.g. neuroblastoma, neurofibromatosis, chordoma, thymoma, adenoid cystic carcinoma, aggressive fibromatosis, myelofibrosis, desmoplastic small cell round tumor and/or the skin, e.g. melanoma and/or the endocrine system, e.g. the thyroid, the adrenal gland,and/or cancer of the blood , e.g. hematological cancer, e.g. leukemia, e.g. acute myeloid leukemia, e.g. chronic myeloid leukemia, e.g. chronic lymphatic leukemia, e.g. acute lymphatic leukemia, e.g. multiple myeloma e.g. lymphomas, and/or myelodysplastic syndrome, systemic mastocytosis, von Hippel-Lindau syndrome, multicentric Castleman disease and/or psioriasis a Hsp90 inhjbitor, which comprises administering a pharmaceutically effective amount of a Hsp90 inhibitor to a human subject about once weekly or more frequently.

Following is a description by example only.

### Example 1: In vitro effects of (R)-2-amino-7-[2-(6-methoxy-pyridin-2-yl)-phenyl]-4-methyl-7,8-dihydro-6H-pyrido[4,3-d]pyrimidin-5-one on a panel of tumor derived cell lines.

Seventeen cancer derived cell lines are used (GTL-16, N87, H1975, A549, H69, A2780, PC3, BT474, SKBR3, MCF-7, A375, MV4;11, K562, KM12L4A, H929, OPM-2 and KMS-11) to test the effect of (R)-2-amino-7-[2-(6-methoxy-pyridin-2-yl)-phenyl]-4-methyl-7,8-dihydro-6H-pyrido[4,3-d]pyrimidin-5-one. These cell lines cover the following 9 tumor types: Gastric, Lung, Ovarian, Prostate, Breast, Melanoma, Leukemia, Colon and Multiple myeloma. After division and medium change, cells from stock culture are seeded on cell plates and cultured for about 18 hours to allow cell growth and attachment before starting the assay. Then on the first day of the assay, (R)-2-amino-7-[2-(6-methoxy-pyridin-2-yl)-phenyl]-4-methyl-7,8-dihydro-6H-pyrido[4,3-d]pyrimidin-5-one is added to the medium at various concentrations. Cells are cultured up to 72 or 96 hours and cell proliferation is determined using commercially available cell proliferation kits.

**Growth inhibition of cancer cell lines by** (R)-2-amino-7-[2-(6-methoxy-pyridin-2-yl)-phenyl]-4-methyl-7,8-dihydro-6H-pyrido[4,3-d]pyrimidin-5-one Table 1 relates to the concentration in nM of (R)-2-amino-7-[2-(6-methoxy-pyridin-2-yl)-phenyl]-4-methyl-7,8-dihydro-6H-pyrido[4,3-d]pyrimidin-5-one which inhibits cell proliferation by 50% (IC₅₀). The cells were continually exposed to (R)-2-amino-7-[2-(6-methoxy-pyridin-2-yl)-phenyl]-4-methyl-7,8-dihydro-6H-pyrido[4,3-d]pyrimidin-5-one for either 72 or 96 hours and cell growth are determined by commercially available kits based on either SRB, Alamar blue, methylene blue or WST-1 methods.

**Table 1**

| **Indication** | **Cell line** | **GI₅₀** |
|---|---|---|
| Gastric | GTL-16 N87 | 18 (N=50) 12 (N=30) |
| Lung | H1975 | 33 (N=8) |
| | A549 | 16 (N=10) |
| | H69 | 37 (N=10) |
| Ovarian | A2780 | 27 (N=14) |
| Prostate | PC3 | 16 (N=2) |
| | BT474 | 6 (N=4) |
| Breast | SKBR3 | 18 (N=7) |
| | MCF-7 | 8 (N=2) |
| Melanoma | A375m | 36 (N=12) |
| Leukemia | MV4;11 | 4 (N=8) |
| | K562 | 24 (N=11) |
| Colon | KM12L4A | 11 (N=12) |
| Multiple myeloma | H929 | 20 (N=4) |
| | OPM-2 | 13 (N=4) |
| | KMS-11 | 7 (N=4) |
| Determination of the anti-proliferative effect of (R)-2-amino-7-[2-(6-methoxy-pyridin-2-yl)-phenyl]-4-methyl-7,8-dihydro-6H-pyrido[4,3-d]pyrimidin-5-one as indicated by the concentration which leads to 50% growth inhibition (GI₅₀). Tumor cell lines were grown in cell culture for 72 hours in the presence of compound. The number of repeats indicated in parentheses. | | |

### Example 2: In vitro effects of (R)-2-amino-7-[2-(6-methoxy-pyridin-2-yl)-phenyl]-4-methyl-7,8-dihydro-6H-pyrido[4,3-d]pyrimidin-5-one on a panel of primary human tumor cells.

The anticancer activity of (R)-2-amino-7-[2-(6-methoxy-pyridin-2-yl)-phenyl]-4-methyl-7,8-dihydro-6H-pyrido[4,3-d]pyrimidin-5-one is evaluated in 30 human tumor xenografts *in vitro* using a clonogenic assay. In this assay, human cells derived from cancer patients are evaluated for the capacity of (R)-2-amino-7-[2-(6-methoxy-pyridin-2-yl)-phenyl]-4-methyl-7,8-dihydro-6H-pyrido[4,3-d]pyrimidin-5-one to inhibit the formation of 3 dimensional colonies. These consist of tumor cells that possess the potential for anchorage independent growth in semisolid medium. The tumor xenografts which have never been cultured in cell culture plastic dishes are isolated from nude mice. Tumor cell suspensions are prepared and incubated in 24 well plates containing layers of soft agar. Under these conditions a special subpopulation of cells selectively grows to colonies. (R)-2-amino-7-[2-(6-methoxy-pyridin-2-yl)-phenyl]-4-methyl-7,8-dihydro-6H-pyrido[4,3-d]pyrimidin-5-one is tested in 6 concentrations up to 1000 nM. The tumor test panel comprises 1 to 10 models of 12 different human tumor types, which were bladder cancer, colon, gastric, non small cell lung (adeno, squamous cell, and large cell), small cell lung, mammary, ovary, and prostate cancer, as well as leukemia, lymphoma, melanoma, and sarcoma. Antitumor effects are recorded as inhibition of colony formation in relation to untreated controls. The concentration which resulted in 50% reduction in colony formation (IC₅₀) is shown in Table 2. Further information on the method has been published (Burger et al., 2004; Fiebig et al., 2004; Smith et al., 2005).

### Growth inhibition of primary human cancer cells by (R)-2-amino-7-[2-(6-methoxy-pyridin-2-yl)-phenyl]-4-methyl-7,8-dihydro-6H-pyrido[4,3-d]pyrimidin-5-one

Table 2 relates to the concentration in nM of (R)-2-amino-7-[2-(6-methoxy-pyridin-2-yl)-phenyl]-4-methyl-7,8-dihydro-6H-pyrido[4,3-d]pyrimidin-5-one which inhibits colony formation by 50% (IC₅₀). The cells are continually exposed to (R)-2-amino-7-[2-(6-methoxy-pyridin-2-yl)-phenyl]-4-methyl-7,8-dihydro-6H-pyrido[4,3-d]pyrimidin-5-one for and colony formation was determined.

**Table 2**

| **Tumor Type** | **Tumor model** | **Histology** | **IC₅₀ (nM)** |
|---|---|---|---|
| Bladder | BXF 1218 | Transitional cell carcinoma | 26 |
| | BXF 1258 | Transitional cell carcinoma | 316 |
| Colon | CXF 1103 | Adenocarcinoma | 27 |
| | CXF 1729 | Papillary adenocarcinoma | 199 |
| | CXF 243 | Adenocarcinoma | 44 |
| Stomach | GXF 1172 | Signet-ring cell carcinoma | 43 |
| | GXF 209 | Signet-ring cell carcinoma | 28 |
| | GXF 214 | Adenocarcinoma | >1000 |
| Leukemia | LEXF CCRFCEM | ALL, T lymphoblast | 27 |
| | LEXFJURKAT | T cell leukemia | 4 |
| | LEXF K562 | Chronic myelogenous leukemia | 13 |
| | LEXF MOLT4 | ALL, T lymphoblast | 21 |
| Lung,non-small cell | LXFA 1012 | Adenocarcinoma | >1000 |
| | LXFA 289 | Adenocarcinoma | 639 |
| | LXFA 526 | Adenocarcinoma | 36 |
| | LXFA 629 | Adenocarcinoma | 27 |
| | LXFA 677 | Adenocarcinoma | 27 |
| | LXFA 737 | Adenocarcinoma | 26 |
| | LXFE 1422 | Squamous cell carcinoma | 20 |
| | LXFE 211 | Squamous cell carcinoma | 0.76 |
| | LXFL 1176 | Mucous large cell lung carcinoma | 21 |
| | LXFL 1647 | Large cell lung carcinoma | 203 |
| Lung, small cell | LXFS 650 | Small cell lung carcinoma | 6 |
| Lymphoma | LYXF U937 | Histiocytic lymphoma | 28 |
| Breast | MAXF 1162 | Invasive ductal carcinoma | 4 |
| | MAXF 1322 | Pap. adenocarcinoma | 52 |
| | MAXF 1384 | Adenocarcinoma | 14 |
| | MAXF 401 | Pap. adenocarcinoma | 15 |
| | MAXF 574 | Carcinoma | 316 |
| | MAXF 583 | Ductual adenocarcinoma | 24 |
| | MAXF 713 | Adenocarcinoma | >1000 |
| | MAXF 857 | Invasive ductal carcinoma | 12 |
| Melanoma | MEXF 1341 | Amelanotic melanoma | >1000 |
| | MEXF 462 | Amelanotic melanoma | 65 |
| | MEXF 989 | Amelanotic melanoma | 17 |
| Ovary | OVXF 1353 | Adenocarcinoma | 53 |
| | OVXF 899 | Pap. serous adenocarcinoma | >1000 |
| Prostate | PRXF 22RV1 | Prostate carcinoma | 23 |
| | PRXF DU145 | Adenocarcinoma | 17 |
| | PRXF MRIH1579 | Adenocarcinoma | >1000 |
| | PRXF PC3M | Adenocarcinoma, metastatic | 278 |
| Sarcoma | SXF 1186 | Osteoblastic osteosarcoma | 21 |
| | SXF 1301 | Malignant rhabdomyosarcoma | 26 |
| | SXF 1410 | Fibroblastic osteosarcoma | >1000 |
| | SXF 463 | Rhabdomyosarcoma | 5 |

The following numbered paragraphs are of special interest.
Paragraph 1: The use of a Hsp90 inhibitor for the manufacture of a pharmaceutical composition for use in the treatment of cancer of the lung,
   the bladder, the colon, the rectum, the liver, the breast, the vagina, the ovaries, the pancreas, the kidney, the stomach, the gastrointestinal tract, the prostate, the head and neck, the skin, the peritoneal cavity, the thyroid, the bone, the brain, the central nervous system, the endocrine system, the thyroid, the adrenal gland and/or the blood and/or myelodysplastic syndrome, systemic mastocytosis, von Hippel-Lindau syndrome, multicentric Castleman disease and/or psioriasis.
Paragraph 2: The use according to paragraph 1 wherein the Hsp90 inhibitor is of Formula (I) 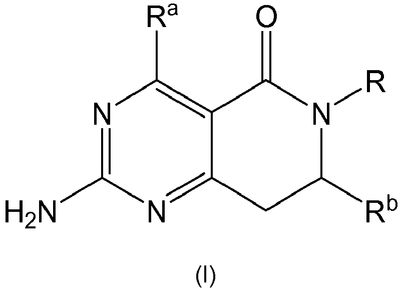 or a stereoisomer, tautomer, pharmaceutically acceptable salt, or prodrug thereof, wherein
   R^{a} is selected from the group consisting of
   (1) hydrogen,
   (2) halogen,
   (3) hydroxyl,
   (4) C₁-C₆ alkoxy,
   (5) thiol,
   (6) C₁₋C₆ alkylthiol,
   (7) substituted or unsubstituted C₁-C₆ alkyl,
   (8) amino or substituted amino,
   (9) substituted or unsubstituted aryl,
   (10) substituted or unsubstituted heteroaryl, and
   (11) substituted or unsubstituted heterocyclyl;
   R is selected from the group consisting of
   (1) hydrogen,
   (2) substituted or unsubstituted C₁-C₆ alkyl,
   (3) substituted or unsubstituted C₂-C₆ alkenyl,
   (4) substituted or unsubstituted C₂-C₆ alkynyl,
   (5) substituted or unsubstituted C₃-C₇ cycloalkyl,
   (6) substituted or unsubstituted C₅-C₇ cycloalkenyl,
   (7) substituted or unsubstituted aryl,
   (8) substituted or unsubstituted heteroaryl, and
   (9) substituted or unsubstituted heterocyclyl;
   R^{b} is selected from the group consisting of
   (1) substituted or unsubstituted C₃-C₇ cycloalkyl,
   (2) substituted or unsubstituted C₅-C₇ cycloalkenyl,
   (3) substituted or unsubstituted aryl,
   (4) substituted or unsubstituted heteroaryl, and
   (5) substituted or unsubstituted heterocyclyl; and with the proviso that when R^{a} is amino, then R^{b} is not phenyl, 4-alkyl-phenyl, 4-alkoxy-phenyl, or 4-halo-phenyl or a pharmaceutically acceptable salt thereof.
Paragraph 3. The use according to Paragraph 1 or 2 wherein the Hsp90 inhibitor is a compound according to formula (III) 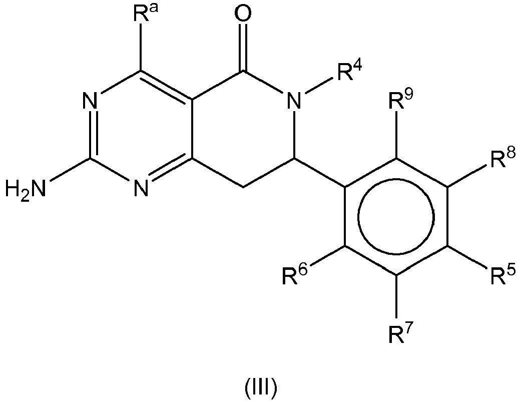 or a stereoisomer, tautomer, pharmaceutically acceptable salt, or prodrug thereof, wherein
   wherein R^{a} is selected from the group consisting of
   (1) hydrogen,
   (2) halogen,
   (3) hydroxyl,
   (4) C₁-C₆ alkoxy,
   (5) thiol,
   (6) C₁₋C₆ alkylthiol,
   (7) substituted or unsubstituted C₁-C₆ alkyl,
   (8) amino or substituted amino,
   (9) substituted or unsubstituted aryl,
   (10) substituted or unsubstituted heteroaryl, and
   (11) substituted or unsubstituted heterocyclyl;
   R⁴ is hydrogen or substituted or unsubstituted C₁-C₆ alkyl;
   R⁵ is hydrogen, alkyl, alkoxy, or halo;
   each of R⁶, R⁷, R⁸, and R⁹ are independently selected from the group consisting of hydrogen, alkyl, alkoxy, halo, substituted or unsubstituted aryl, and substituted or unsubstituted heteroaryl;
   with the proviso that when R^{a} is amino and R⁶, R⁷, R³, and R⁹ are hydrogen, then R⁵ is not hydrogen, alkyl, alkoxy, or halo or a pharmaceutically acceptable salt thereof
Paragraph 4. A Hsp90 inhibitor for use in the treatment of cancer of the lung, bladder, the colon, the rectum, the liver, the breast, the vagina, the ovaries, the pancreas, the kidney, the stomach, the gastrointestinal tract, the prostate, the head and neck, the skin, the peritoneal cavity, the thyroid, the bone, the brain, the central nervous system, the endocrine system, the thyroid, the adrenal gland and/or the blood and/or myelodysplastic syndrome, systemic mastocytosis, von Hippel-Lindau syndrome, multicentric Castleman disease and/or psioriasis;
   wherein the lung cancer is selected from non small cell lung cancer, small cell lung cancer and pleural mesothelioma.
Paragraph 5. The Hsp90 inhibitor for use according to Paragraph 4 wherein the Hsp90 inhibitor is of Formula (I) 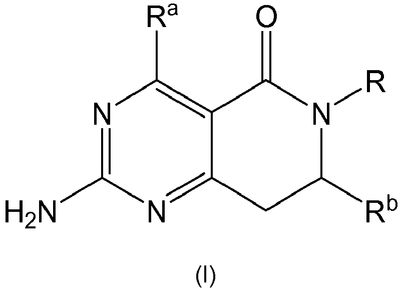 or a stereoisomer, tautomer, pharmaceutically acceptable salt, or prodrug thereof, wherein
   R^{a} is selected from the group consisting of
   (1) hydrogen,
   (2) halogen,
   (3) hydroxyl,
   (4) C₁-C₆ alkoxy,
   (5) thiol,
   (6) C₁-C₆ alkylthiol,
   (7) substituted or unsubstituted C₁-C₆ alkyl,
   (8) amino or substituted amino,
   (9) substituted or unsubstituted aryl,
   (10) substituted or unsubstituted heteroaryl, and
   (11) substituted or unsubstituted heterocyclyl;
   R is selected from the group consisting of
   (1) hydrogen,
   (2) substituted or unsubstituted C₁-C₆ alkyl,
   (3) substituted or unsubstituted C₂-C₆ alkenyl,
   (4) substituted or unsubstituted C₂-C₆ alkynyl,
   (5) substituted or unsubstituted C₃-C₇ cycloalkyl,
   (6) substituted or unsubstituted C₅-C₇ cycloalkenyl,
   (7) substituted or unsubstituted aryl,
   (8) substituted or unsubstituted heteroaryl, and
   (9) substituted or unsubstituted heterocyclyl;
   R^{b} is selected from the group consisting of
   (1) substituted or unsubstituted C₃-C₇ cycloalkyl,
   (2) substituted or unsubstituted C₅-C₇ cycloalkenyl,
   (3) substituted or unsubstituted aryl,
   (4) substituted or unsubstituted heteroaryl, and
   (5) substituted or unsubstituted heterocyclyl; and
   with the proviso that when R^{a} is amino, then R^{b} is not phenyl, 4-alkyl-phenyl, 4-alkoxy-phenyl, or 4-halo-phenyl or an acceptable salt thereof.
Paragraph 6. The Hsp90 inhibitor for use according to Paragraph 4 or 5 wherein the Hsp90 inhibitor is
   a compound according to formula (III) 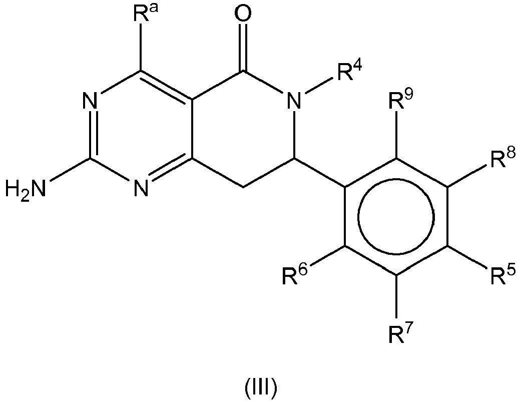 or a stereoisomer, tautomer, pharmaceutically acceptable salt, or prodrug thereof, wherein
   wherein R^{a} is selected from the group consisting of
   (1) hydrogen,
   (2) halogen,
   (3) hydroxyl,
   (4) C₁-C₆ alkoxy,
   (5) thiol,
   (6) C₁-C₆ alkylthiol,
   (7) substituted or unsubstituted C₁-C₆ alkyl,
   (8) amino or substituted amino,
   (9) substituted or unsubstituted aryl,
   (10) substituted or unsubstituted heteroaryl, and
   (11) substituted or unsubstituted heterocyclyl;
   R⁴ is hydrogen or substituted or unsubstituted C₁-C₆ alkyl;
   R⁵ is hydrogen, alkyl, alkoxy, or halo;
   each of R⁶, R⁷, R⁸, and R⁹ are independently selected from the group consisting of hydrogen, alkyl, alkoxy, halo, substituted or unsubstituted aryl, and substituted or unsubstituted heteroaryl;
   with the proviso that when R^{a} is amino and R⁶, R⁷, R⁸, and R⁹ are hydrogen, then R⁵ is not hydrogen, alkyl, alkoxy, or halo;
Paragraph 7. The use of Paragraph 2, wherein the Hsp90 inhibitor is
   (R)-2-amino-7-[2-(2-fluoro-pyridin-3-yl)-phenyl]-4-methyl-7,8-dihydro-6H-pyrido[4,3-d]pyrimidin-5-one,
   (S)-2-amino-6-benzyl-7-[4-fluoro-2-(2-fluoro-pyridin-3-yl)-phenyl]-4-methyl-7,8-dihydro-6H-pyrido[4,3-d]pyrimidin-5-one,
   (R)-2-amino-7-[4-fluoro-2-(2-fluoro-pyridin-3-yl)-phenyl]-4-methyl-7,8-dihydro-6H-pyrido[4,3-d]pyrimidin-5-one,
   (R)-2-amino-7-(2-bromo-4-fluoro-phenyl)-6-[(S)-1-(4-methoxy-phenyl)-ethyl]-4-methyl-7,8-dihydro-6H-pyrido[4,3-d]pyrimidin-5-one,
   (R)-2-amino-7-[2-(6-methoxy-pyridin-2-yl)-phenyl]-4-methyl-7,8-dihydro-6H-pyrido[4,3-d]pyrimidin-5-one,
   (R)-2-amino-7-[4-fluoro-2-(6-methoxy-pyridin-2-yl)-phenyl]-4-methyl-7,8-dihydro-6H-pyrido[4,3-d]pyrimidin-5-one,
   2-amino-7-[4-fluoro-2-(6-methoxy-pyridin-2-yl)-phenyl]-4,6-dimethyl-7,8-dihydro-6H-pyrido[4,3-d]pyrimidin-5-one,
   2-amino-7-[4-fluoro-2-(2-fluoro-pyridin-3-yl)-phenyl]-4,6-dimethyl-7,8-dihydro-6H-pyrido[4,3-d]pyrimidin-5-one,
   2-amino-7-[4-fluoro-2-(6-methoxypyridin-2-yl)phenyl]-4-methyl-7,8-dihydropyrido[4,3-*d*]pyrimidin-5(6*H*)-one,
   2-amino-7-[2-(6-methoxy-pyrazin-2-yl)-phenyl]-4-methyl-7,8-dihydro-6H-pyrido[4,3-d]pyrimidin-5-one,
   (R)-2-amino-7-[4-fluoro-2-(6-methoxy-pyrazin-2-yl)-phenyl]-4-methyl-7,8-dihydro-6H-pyrido[4,3-d]pyrimidin-5-one,
   2-amino-7-[4-fluoro-2-(6-methoxy-pyrazin-2-yl)-phenyl]-4,6-dimethyl-7,8-dihydro-6H-pyrido[4,3-d]pyrimidin-5-one,
   2-amino-7-[2-(2-methoxy-pyridin-3-yl)-phenyl]-4-methyl-7,8-dihydro-6H-pyrido[4,3-d]pyrimidin-5-one,
   2-amino-7-(5,2'-difluoro-biphenyl-2-yl)-4-methyl-7, 8-dihydro-6H-pyrido[4,3-d]pyrimidin-5-one,
   2-amino-7-(5-fluoro-2'-trifluoromethoxy-biphenyl-2-yl)-4-methyl-7, 8-dihydro-6H-pyrido[4,3-d]pyrimidin-5-one,
   2-amino-7-[2-(2-chloro-pyridin-3-yl)-4-fluoro-phenyl]-4-methyl-7,8-dihydro-6H-pyrido[4,3-d]pyrimidin-5-one,
   2-amino-7-[4-fluoro-2-(6-fluoro-pyridin-3-yl)-phenyl]-4-methyl-7,8-dihydro-6H-pyrido[4,3-d]pyrimidin-5-one,
   2-amino-7-(4-fluoro-2-isoquinolin-4-yl-phenyl)-4-methyl-7,8-dihydro-6H-pyrido[4,3-d]pyrimidin-5-one,
   2-amino-7-(5,3'-difluoro-biphenyl-2-yl)-4-methyl-7,8-dihydro-6H-pyrido[4,3-d]pyrimidin-5-one,
   2-amino-7-[2-(4-chloro-pyridin-3-yl)-4-fluoro-phenyl]-4-methyl-7,8-dihydro-6H-pyrido[4,3-d]pyrimidin-5-one,
   2-amino-7-(5,2'-difluoro-3'-methoxy-biphenyl-2-yl)-4-methyl-7,8-dihydro-6H-pyrido[4,3-d]pyrimidin-5-one,
   2-amino-7-(5,4'-difluoro-2'-methyl-biphenyl-2-yl)-4-methyl-7,8-dihydro-6H-pyrido[4,3-d]pyrimidin-5-one,
   2-amino-7-(5-fluoro-2'-methoxy-biphenyl-2-yl)-4-methyl-7,8-dihydro-6H-pyrido[4,3-d]pyrimidin-5-one,
   2-amino-7-(4-fluoro-2-pyrimidin-5-yl-phenyl)-4-methyl-7,8-dihydro-6H-pyrido[4,3-d]pyrimidin-5-one,
   2-amino-7-[4-fluoro-2-(2-methoxy-pyridin-3-yl)-phenyl]-4-methyl-7,8-dihydro-6H-pyrido[4,3-d]pyrimidin-5-one,
   2-amino-7-(5-fluoro-3'-methoxy-biphenyl-2-yl)-4-methyl-7,8-dihydro-6H-pyrido[4,3-d]pyrimidin-5-one,
   (R)-2-amino-6-(3-amino-propyl)-7-[4-fluoro-2-(6-methoxy-pyridin-2-yl)-phenyl]-4-methyl-7,8-dihydro-6H-pyrido[4,3-d]pyrimidin-5-one,
   2-amino-7-(4-fluoro-2-pyridin-3-yl-phenyl)-4-methyl-7,8-dihydro-6H-pyrido[4,3-d]pyrimidin-5-one,
   2-amino-7-(5,2'-difluoro-4'-methyl-biphenyl-2-yl)-4-methyl-7,8-dihydro-6H-pyrido[4,3-d]pyrimidin-5-one,
   2-amino-7-[4-fluoro-2-(1-methyl-1 H-pyrazol-4-yl)-phenyl]-4-methyl-7,8-dihydro-6H-pyrido[4,3-d]pyrimidin-5-one,
   2-amino-7-[4-fluoro-2-(1H-pyrazol-4-yl)-phenyl]-4-methyl-7,8-dihydro-6H-pyrido[4,3-d]pyrimidin-5-one,
   2-amino-4-methyl-7-(5,2',3'-trifluoro-biphenyl-2-yl)-7,8-dihydro-6H-pyrido[4,3-d]pyrimidin-5-one,
   2-amino-7-(2-bromo-4-fluoro-phenyl)-4-methyl-6-(2-methyl-2-morpholin-4-yl-propyl)-7,8-dihydro-6H-pyrido[4,3-d]pyrimidin-5-one,
   2-amino-7-(3'-dimethylamino-5-fluoro-biphenyl-2-yl)-4-methyl-7,8-dihydro-6H-pyrido[4,3-d]pyrimidin-5-one,
   2-amino-7-[2-(2,4-dimethoxy-pyrimidin-5-yl)-4-fluoro-phenyl]-4-methyl-7,8-dihydro-6H-pyrido[4,3-d]pyrimidin-5-one,
   2-amino-7-[4-fluoro-2-(5-methoxy-pyridin-3-yl)-phenyl]-4-methyl-7,8-dihydro-6H-pyrido[4,3-d]pyrimidin-5-one,
   2-amino-7-(4-fluoro-2-pyrimidin-5-yl-phenyl)-4-methyl-6-(2-methyl-2-morpholin-4-yl-propyl)-7,8-dihydro-6H-pyrido[4,3-d]pyrimidin-5-one,
   2-amino-7-[4-fluoro-2-(2-methoxy-pyridin-3-yl)-phenyl]-4-methyl-6-(2-methyl-2-morpholin-4-yl-propyl)-7,8-dihydro-6H-pyrido[4,3-d]pyrimidin-5-one,
   2-amino-7-(5-fluoro-3'-methoxy-biphenyl-2-yl)-4-methyl-6-(2-methyl-2-morpholin-4-yl-propyl)-7,8-dihydro-6H-pyrido[4,3-d]pyrimidin-5-one,
   (R)-2-amino-7-[4-fluoro-2-(4-methoxy-5-methyl-pyrimidin-2-yl)-phenyl]-4-methyl-7,8-dihydro-6H-pyrido[4,3-d]pyrimidin-5-one,
   2-amino-7-(4-fluoro-2-furan-3-yl-phenyl)-4-methyl-7,8-dihydro-6H-pyrido[4,3-d]pyrimidin-5-one, or
   a pharmaceutically acceptable salt or prodrug thereof.
Paragraph 8. The use of Paragraph 7, wherein the Hsp90 inhibitor is (R)-2-amino-7-[4-fluoro-2-(6-methoxy-pyridin-2-yl)-phenyl]-4-methyl-7,8-dihydro-6H-pyrido[4,3-d]pyrimidin-5-one, or a pharmaceutically acceptable salt or prodrug thereof.
Paragraph 9. The use of Paragraph 8, wherein the Hsp90 inhibitor is
   (R)-2-amino-7-[2-(2-fluoro-pyridin-3-yl)-phenyl]-4-methyl-7,8-dihydro-6H-pyrido[4,3-d]pyrimidin-5-one,
   (S)-2-amino-6-benzyl-7-[4-fluoro-2-(2-fluoro-pyridin-3-yl)-phenyl]-4-methyl-7,8-dihydro-6H-pyrido[4,3-d]pyrimidin-5-one,
   (R)-2-amino-7-[4-fluoro-2-(2-fluoro-pyridin-3-yl)-phenyl]-4-methyl-7,8-dihydro-6H-pyrido[4,3-d]pyrimidin-5-one,
   (R)-2-amino-7-(2-bromo-4-fluoro-phenyl)-6-[(S)-1 -(4-methoxy-phenyl)-ethyl]-4-methyl-7,8-dihydro-6H-pyrido[4,3-d]pyrimidin-5-one,
   (R)-2-amino-7-[2-(6-methoxy-pyridin-2-yl)-phenyl]-4-methyl-7,8-dihydro-6H-pyrido[4,3-d]pyrimidin-5-one,
   (R)-2-amino-7-[4-fluoro-2-(6-methoxy-pyridin-2-yl)-phenyl]-4-methyl-7,8-dihydro-6H-pyrido[4,3-d]pyrimidin-5-one,
   2-amino-7-[4-fluoro-2-(6-methoxy-pyridin-2-yl)-phenyl]-4,6-dimethyl-7,8-dihydro-6H-pyrido[4,3-d]pyrimidin-5-one,
   2-amino-7-[4-fluoro-2-(2-fluoro-pyridin-3-yl)-phenyl]-4,6-dimethyl-7,8-dihydro-6H-pyrido[4,3-d]pyrimidin-5-one,
   2-amino-7-[4-fluoro-2-(6-methoxypyridin-2-yl)phenyl]-4-methyl-7,8-dihydropyrido[4,3-*d*]pyrimidin-5(6H)-one,
   2-amino-7-[2-(6-methoxy-pyrazin-2-yl)-phenyl]-4-methyl-7,8-dihydro-6H-pyrido[4,3-d]pyrimidin-5-one,
   (R)-2-amino-7-[4-fluoro-2-(6-methoxy-pyrazin-2-yl)-phenyl]-4-methyl-7,8-dihydro-6H-pyrido[4,3-d]pyrimidin-5-one,
   2-amino-7-[4-fluoro-2-(6-methoxy-pyrazin-2-yl)-phenyl]-4,6-dimethyl-7,8-dihydro-6H-pyrido[4,3-d]pyrimidin-5-one,
   2-amino-7-[2-(2-methoxy-pyridin-3-yl)-phenyl]-4-methyl-7,8-dihydro-6H-pyrido[4,3-d]pyrimidin-5-one,
   2-amino-7-(5,2'-difluoro-biphenyl-2-yl)-4-methyl-7,8-dihydro-6H-pyrido[4,3-d]pyrimidin-5-one,
   2-amino-7-(5-fluoro-2'-trifluoromethoxy-biphenyl-2-yl)-4-methyl-7,8-dihydro-6H-pyrido[4,3-d]pyrimidin-5-one,
   2-amino-7-[2-(2-chloro-pyridin-3-yl)-4-fluoro-phenyl]-4-methyl-7,8-dihydro-6H-pyrido[4,3-d]pyrimidin-5-one,
   2-amino-7-[4-fluoro-2-(6-fluoro-pyridin-3-yl)-phenyl]-4-methyl-7,8-dihydro-6H-pyrido[4,3-d]pyrimidin-5-one,
   2-amino-7-(4-fluoro-2-isoquinolin-4-yl-phenyl)-4-methyl-7,8-dihydro-6H-pyrido[4,3-d]pyrimidin-5-one,
   2-amino-7-(5,3'-difluoro-biphenyl-2-yl)-4-methyl-7,8-dihydro-6H-pyrido[4,3-d]pyrimidin-5-one,
   2-amino-7-[2-(4-chloro-pyridin-3-yl)-4-fluoro-phenyl]-4-methyl-7,8-dihydro-6H-pyrido[4,3-d]pyrimidin-5-one,
   2-amino-7-(5,2'-difluoro-3'-methoxy-biphenyl-2-yl)-4-methyl-7,8-dihydro-6H-pyrido[4,3-d]pyrimidin-5-one,
   2-amino-7-(5,4'-difluoro-2'-methyl-biphenyl-2-yl)-4-methyl-7,8-dihydro-6H-pyrido[4,3-d]pyrimidin-5-one,
   2-amino-7-(5-fluoro-2'-methoxy-biphenyl-2-yl)-4-methyl-7,8-dihydro-6H-pyrido[4,3-d]pyrimidin-5-one,
   2-amino-7-(4-fluoro-2-pyrimidin-5-yl-phenyl)-4-methyl-7,8-dihydro-6H-pyrido[4,3-d]pyrimidin-5-one,
   2-amino-7-[4-fluoro-2-(2-methoxy-pyridin-3-yl)-phenyl]-4-methyl-7,8-dihydro-6H-pyrido[4,3-d]pyrimidin-5-one,
   2-amino-7-(5-fluoro-3'-methoxy-biphenyl-2-yl)-4-methyl-7,8-dihydro-6H-pyrido[4,3-d]pyrimidin-5-one,
   (R)-2-amino-6-(3-amino-propyl)-7-[4-fluoro-2-(6-methoxy-pyridin-2-yl)-phenyl]-4-methyl-7,8-dihydro-6H-pyrido[4,3-d]pyrimidin-5-one,
   2-amino-7-(4-fluoro-2-pyridin-3-yl-phenyl)-4-methyl-7,8-dihydro-6H-pyrido[4,3-d]pyrimidin-5-one,
   2-amino-7-(5,2'-difluoro-4'-methyl-biphenyl-2-yl)-4-methyl-7,8-dihydro-6H-pyrido[4,3-d]pyrimidin-5-one,
   2-amino-7-[4-fluoro-2-(1-methyl-1H-pyrazol-4-yl)-phenyl]-4-methyl-7,8-dihydro-6H-pyrido[4,3-d]pyrimidin-5-one,
   2-amino-7-[4-fluoro-2-(1H-pyrazol-4-yl)-phenyl]-4-methyl-7,8-dihydro-6H-pyrido[4,3-d]pyrimidin-5-one,
   2-amino-4-methyl-7-(5,2',3'-trifiuoro-biphenyl-2-yl)-7,8-dihydro-6H-pyrido[4,3-d]pyrimidin-5-one,
   2-amino-7-(2-bromo-4-fluoro-phenyl)-4-methyl-6-(2-methyl-2-morpholin-4-yl-propyl)-7,8-dihydro-6H-pyrido[4,3-d]pyrimidin-5-one,
   2-amino-7-(3'-dimethylamino-5-fluoro-biphenyl-2-yl)-4-methyl-7,8-dihydro-6H-pyrido[4,3-d]pyrimidin-5-one,
   2-amino-7-[2-(2,4-dimethoxy-pyrimidin-5-yl)-4-fluoro-phenyl]-4-methyl-7,8-dihydro-6H-pyrido[4,3-d]pyrimidin-5-one,
   2-amino-7-[4-fluoro-2-(5-methoxy-pyridin-3-yl)-phenyl]-4-methyl-7,8-dihydro-6H-pyrido[4,3-d]pyrimidin-5-one,
   2-amino-7-(4-fluoro-2-pyrimidin-5-yl-phenyl)-4-methyl-6-(2-methyl-2-morpholin-4-yl-propyl)-7,8-dihydro-6H-pyrido[4,3-d]pyrimidin-5-one,
   2-amino-7-[4-fluoro-2-(2-methoxy-pyridin-3-yl)-phenyl]-4-methyl-6-(2-methyl-2-morpholin-4-yl-propyl)-7,8-dihydro-6H-pyrido[4,3-d]pyrimidin-5-one,
   2-amino-7-(5-fluoro-3'-methoxy-biphenyl-2-yl)-4-methyl-6-(2-methyl-2-morpholin-4-yl-propyl)-7,8-dihydro-6H-pyrido[4,3-d]pyrimidin-5-one,
   (R)-2-amino-7-[4-fluoro-2-(4-methoxy-5-methyl-pyrimidin-2-yl)-phenyl]-4-methyl-7,8-dihydro-6H-pyrido[4,3-d]pyrimidin-5-one,
   2-amino-7-(4-fluoro-2-furan-3-yl-phenyl)-4-methyl-7,8-dihydro-6H-pyrido[4,3-d]pyrimidin-5-one, or
   a pharmaceutically acceptable salt or prodrug thereof.
Paragraph 10. The use of Paragraph 9, wherein the Hsp90 inhibitor is (R)-2-amino-7-[4-fluoro-2-(6-methoxy-pyridin-2-yl)-phenyl]-4-methyl-7,8-dihydro-6H-pyrido[4,3-d]pyrimidin-5-one, or a pharmaceutically acceptable salt or prodrug thereof.

## Claims

1. The use of a Hsp90 inhibitor for the manufacture of a pharmaceutical composition for the treatment of lung cancer,
wherein the lung cancer is selected from non small cell lung cancer, small cell lung cancer and pleural mesothelioma.

2. The use according to claim 1 wherein the Hsp90 inhibitor is of Formula (I) 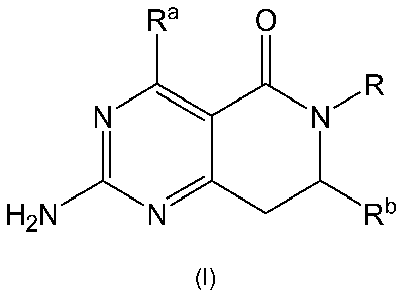 or a stereoisomer, tautomer, pharmaceutically acceptable salt, or prodrug thereof, wherein
R^{a} is selected from the group consisting of
(1) hydrogen,
(2) halogen,
(3) hydroxyl,
(4) C₁-C₆ alkoxy,
(5) thiol,
(6) C₁₋C₆ alkylthiol,
(7) substituted or unsubstituted C₁-C₆ alkyl,
(8) amino or substituted amino,
(9) substituted or unsubstituted aryl,
(10) substituted or unsubstituted heteroaryl, and
(11) substituted or unsubstituted heterocyclyl;
R is selected from the group consisting of
(1) hydrogen,
(2) substituted or unsubstituted C₁-C₆ alkyl,
(3) substituted or unsubstituted C₂-C₆ alkenyl,
(4) substituted or unsubstituted C₂-C₆ alkynyl,
(5) substituted or unsubstituted C₃-C₇ cycloalkyl,
(6) substituted or unsubstituted C₅-C₇ cycloalkenyl,
(7) substituted or unsubstituted aryl,
(8) substituted or unsubstituted heteroaryl, and
(9) substituted or unsubstituted heterocyclyl;
R^{b} is selected from the group consisting of
(1) substituted or unsubstituted C₃-C₇ cycloalkyl,
(2) substituted or unsubstituted C₅-C₇ cycloalkenyl,
(3) substituted or unsubstituted aryl,
(4) substituted or unsubstituted heteroaryl, and
(5) substituted or unsubstituted heterocyclyl; and
with the proviso that when R^{a} is amino, then R^{b} is not phenyl, 4-alkyl-phenyl, 4-alkoxy-phenyl, or 4-halo-phenyl or a pharmaceutically acceptable salt thereof.

3. The use according to claim 1 or 2 wherein the Hsp90 inhibitor is a compound according to formula (III) 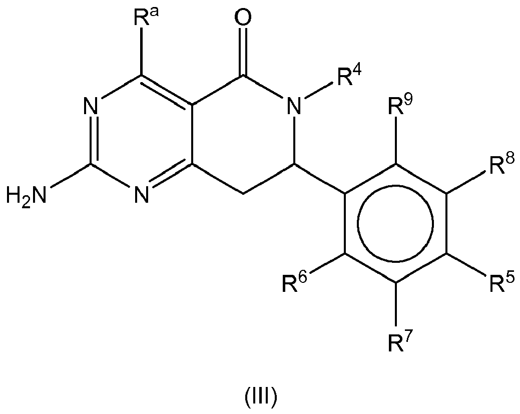 or a stereoisomer, tautomer, pharmaceutically acceptable salt, or prodrug thereof, wherein
wherein R^{a} is selected from the group consisting of
(1) hydrogen,
(2) halogen,
(3) hydroxyl,
(4) C₁-C₆ alkoxy,
(5) thiol,
(6) C₁₋C₆ alkylthiol,
(7) substituted or unsubstituted C₁-C₆ alkyl,
(8) amino or substituted amino,
(9) substituted or unsubstituted aryl,
(10) substituted or unsubstituted heteroaryl, and
(11) substituted or unsubstituted heterocyclyl;
R⁴ is hydrogen or substituted or unsubstituted C₁-C₆ alkyl;
R⁵ is hydrogen, alkyl, alkoxy, or halo;
each of R⁶, R⁷, R⁸, and R⁹ are independently selected from the group consisting of hydrogen, alkyl, alkoxy, halo, substituted or unsubstituted aryl, and substituted or unsubstituted heteroaryl;
with the proviso that when R^{a} is amino and R⁶, R⁷, R⁸, and R⁹ are hydrogen, then R⁵ is not hydrogen, alkyl, alkoxy, or halo or a pharmaceutically acceptable salt thereof

4. A Hsp90 inhibitor for use in the treatment of lung cancer,
wherein the lung cancer is selected from non small cell lung cancer, small cell lung cancer and pleural mesothelioma.

5. The Hsp90 inhibitor for use according to claim 4 wherein the Hsp90 inhibitor is of Formula (I) 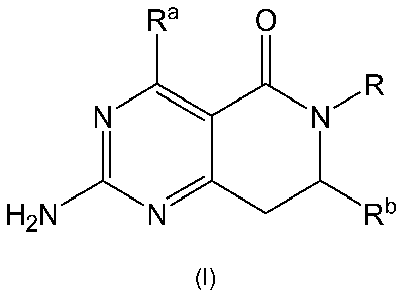 or a stereoisomer, tautomer, pharmaceutically acceptable salt, or prodrug thereof, wherein
R^{a} is selected from the group consisting of
(1) hydrogen,
(2) halogen,
(3) hydroxyl,
(4) C₁-C₆ alkoxy,
(5) thiol,
(6) C₁-C₆ alkylthiol,
(7) substituted or unsubstituted C₁-C₆ alkyl,
(8) amino or substituted amino,
(9) substituted or unsubstituted aryl,
(10) substituted or unsubstituted heteroaryl, and
(11) substituted or unsubstituted heterocyclyl;
R is selected from the group consisting of
(1) hydrogen,
(2) substituted or unsubstituted C₁-C₆ alkyl,
(3) substituted or unsubstituted C₂-C₆ alkenyl,
(4) substituted or unsubstituted C₂-C₆ alkynyl,
(5) substituted or unsubstituted C₃-C₇ cycloalkyl,
(6) substituted or unsubstituted C₅-C₇ cycloalkenyl,
(7) substituted or unsubstituted aryl,
(8) substituted or unsubstituted heteroaryl, and
(9) substituted or unsubstituted heterocyclyl;
R^{b} is selected from the group consisting of
(1) substituted or unsubstituted C₃-C₇ cycloalkyl,
(2) substituted or unsubstituted C₅-C₇ cycloalkenyl,
(3) substituted or unsubstituted aryl,
(4) substituted or unsubstituted heteroaryl, and
(5) substituted or unsubstituted heterocyclyl; and with the proviso that when R^{a} is amino, then R^{b} is not phenyl, 4-alkyl-phenyl, 4-alkoxy-phenyl, or 4-halo-phenyl or an acceptable salt thereof.

6. The Hsp90 inhibitor for use according to claim 4 or 5 wherein the Hsp90 inhibitor is
a compound according to formula (III) 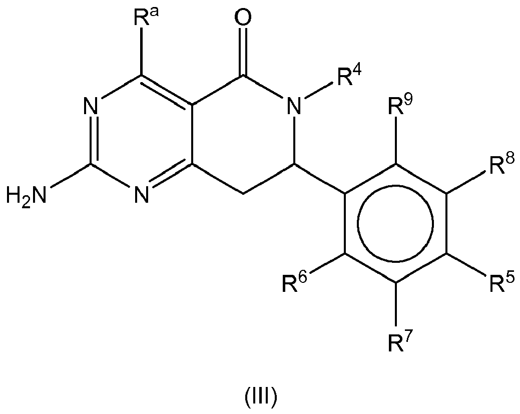 or a stereoisomer, tautomer, pharmaceutically acceptable salt, or prodrug thereof, wherein
wherein R^{a} is selected from the group consisting of
(1) hydrogen,
(2) halogen,
(3) hydroxyl,
(4) C₁-C₆ alkoxy,
(5) thiol,
(6) C₁-C₆ alkylthiol,
(7) substituted or unsubstituted C₁-C₆ alkyl,
(8) amino or substituted amino,
(9) substituted or unsubstituted aryl,
(10) substituted or unsubstituted heteroaryl, and
(11) substituted or unsubstituted heterocyclyl;
R⁴ is hydrogen or substituted or unsubstituted C₁-C₆ alkyl;
R⁵ is hydrogen, alkyl, alkoxy, or halo;
each of R⁶, R⁷, R⁸, and R⁹ are independently selected from the group consisting of hydrogen, alkyl, alkoxy, halo, substituted or unsubstituted aryl, and substituted or unsubstituted heteroaryl;
with the proviso that when R^{a} is amino and R⁶, R⁷, R⁸, and R⁹ are hydrogen, then R⁵ is not hydrogen, alkyl, alkoxy, or halo;

7. The use of claim 2, wherein the Hsp90 inhibitor is
(R)-2-amino-7-[2-(2-fluoro-pyridin-3-yl)-phenyl]-4-methyl-7,8-dihydro-6H-pyrido[4,3-d]pyrimidin-5-one,
(S)-2-amino-6-benzyl-7-[4-fluoro-2-(2-fluoro-pyridin-3-yl)-phenyl]-4-methyl-7,8-dihydro-6H-pyrido[4,3-d]pyrimidin-5-one,
(R)-2-amino-7-[4-fluoro-2-(2-fluoro-pyridin-3-yl)-phenyl]-4-methyl-7,8-dihydro-6H-pyrido[4,3-d]pyrimidin-5-one,
(R)-2-amino-7-(2-bromo-4-fluoro-phenyl)-6-[(S)-1-(4-methoxy-phenyl)-ethyl]-4-methyl-7,8-dihydro-6H-pyrido[4,3-d]pyrimidin-5-one,
(R)-2-amino-7-[2-(6-methoxy-pyridin-2-yl)-phenyl]-4-methyl-7,8-dihydro-6H-pyrido[4,3-d]pyrimidin-5-one,
(R)-2-amino-7-[4-fluoro-2-(6-methoxy-pyridin-2-yl)-phenyl]-4-methyl-7,8-dihydro-6H-pyrido[4,3-d]pyrimidin-5-one,
2-amino-7-[4-fluoro-2-(6-methoxy-pyridin-2-yl)-phenyl]-4,6-dimethyl-7,8-dihydro-6H-pyrido[4,3-d]pyrimidin-5-one,
2-amino-7-[4-fluoro-2-(2-fluoro-pyridin-3-yl)-phenyl]-4,6-dimethyl-7,8-dihydro-6H-pyrido[4,3-d]pyrimidin-5-one,
2-amino-7-[4-fluoro-2-(6-methoxypyridin-2-yl)phenyl]-4-methyl-7,8-dihydropyrido[4,3-*d*]pyrimidin-5(6*H*)-one,
2-amino-7-[2-(6-methoxy-pyrazin-2-yl)-phenyl]-4-methyl-7,8-dihydro-6H-pyrido[4,3-d]pyrimidin-5-one,
(R)-2-amino-7-[4-fluoro-2-(6-methoxy-pyrazin-2-yl)-phenyl]-4-methyl-7,8-dihydro-6H-pyrido[4,3-d]pyrimidin-5-one,
2-amino-7-[4-fluoro-2-(6-methoxy-pyrazin-2-yl)-phenyl]-4,6-dimethyl-7,8-dihydro-6H-pyrido[4,3-d]pyrimidin-5-one,
2-amino-7-[2-(2-methoxy-pyridin-3-yl)-phenyl]-4-methyl-7,8-dihydro-6H-pyrido[4,3-d]pyrimidin-5-one,
2-amino-7-(5,2'-difluoro-biphenyl-2-yl)-4-methyl-7,8-dihydro-6H-pyrido[4,3-d]pyrimidin-5-one,
2-amino-7-(5-fluoro-2'-trifluoromethoxy-biphenyl-2-yl)-4-methyl-7,8-dihydro-6 H-pyrido[4,3-d]pyrimidin-5-one,
2-amino-7-[2-(2-chloro-pyridin-3-yl)-4-fluoro-phenyl]-4-methyl-7,8-d ihydro-6 H-pyrido[4,3-d]pyrimidin-5-one,
2-amino-7-[4-fluoro-2-(6-fluoro-pyridin-3-yl)-phenyl]-4-methyl-7,8-dihydro-6H-pyrido[4,3-d]pyrimidin-5-one,
2-amino-7-(4-fluoro-2-isoquinolin-4-yl-phenyl)-4-methyl-7,8-dihydro-6H-pyrido[4,3-d]pyrimidin-5-one,
2-amino-7-(5,3'-difluoro-biphenyl-2-yl)-4-methyl-7,8-dihydro-6H-pyrido[4,3-d]pyrimidin-5-one,
2-amino-7-[2-(4-chloro-pyridin-3-yl)-4-fluoro-phenyl]-4-methyl-7,8-dihydro-6H-pyrido[4,3-d]pyrimidin-5-one,
2-amino-7-(5,2'-difluoro-3'-methoxy-biphenyl-2-yl)-4-methyl-7,8-dihydro-6H-pyrido[4,3-d]pyrimidin-5-one,
2-amino-7-(5,4'-difluoro-2'-methyl-biphenyl-2-yl)-4-methyl-7,8-dihydro-6 H-pyrido[4,3-d]pyrimidin-5-one,
2-amino-7-(5-fluoro-2'-methoxy-biphenyl-2-yl)-4-methyl-7,8-dihydro-6H-pyrido[4,3-d]pyrimidin-5-one,
2-amino-7-(4-fluoro-2-pyrimidin-5-yl-phenyl)-4-methyl-7,8-dihydro-6H-pyrido[4,3-d]pyrimidin-5-one,
2-amino-7-[4-fluoro-2-(2-methoxy-pyridin-3-yl)-phenyl]-4-methyl-7,8-dihydro-6H-pyrido[4,3-d]pyrimidin-5-one,
2-amino-7-(5-fluoro-3'-methoxy-biphenyl-2-yl)-4-methyl-7,8-dihydro-6H-pyrido[4,3-d]pyrimidin-5-one,
(R)-2-amino-6-(3-amino-propyl)-7-[4-fluoro-2-(6-methoxy-pyridin-2-yl)-phenyl]-4-methyl-7,8-dihydro-6H-pyrido[4,3-d]pyrimidin-5-one,
2-amino-7-(4-fluoro-2-pyridin-3-yl-phenyl)-4-methyl-7,8-dihydro-6H-pyrido[4,3-d]pyrimidin-5-one,
2-amino-7-(5,2'-difluoro-4'-methyl-biphenyl-2-yl)-4-methyl-7,8-dihydro-6H-pyrido[4,3-d]pyrimidin-5-one,
2-amino-7-[4-fluoro-2-(1-methyl-1H-pyrazol-4-yl)-phenyl]-4-methyl-7,8-dihydro-6H-pyrido[4,3-d]pyrimidin-5-one,
2-amino-7-[4-fl uoro-2-(1H-pyrazol-4-yl)-phenyl]-4-methyl-7,8-dihydro-6H-pyrido[4,3-d]pyrimidin-5-one,
2-amino-4-methyl-7-(5,2',3'-trifluoro-biphenyl-2-yl)-7,8-dihydro-6H-pyrido[4,3-d]pyrimidin-5-one,
2-amino-7-(2-bromo-4-fluoro-phenyl)-4-methyl-6-(2-methyl-2-morpholin-4-yl-propyl)-7,8-dihydro-6H-pyrido[4,3-d]pyrimidin-5-one,
2-amino-7-(3'-dimethylamino-5-fluoro-biphenyl-2-yl)-4-methyl-7,8-dihydro-6H-pyrido[4,3-d]pyrimidin-5-one,
2-amino-7-[2-(2,4-dimethoxy-pyrimidin-5-yl)-4-fluoro-phenyl]-4-methyl-7,8-dihydro-6H-pyrido[4,3-d]pyrimidin-5-one,
2-amino-7-[4-fluoro-2-(5-methoxy-pyridin-3-yl)-phenyl]-4-methyl-7,8-dihydro-6H-pyrido[4,3-d]pyrimidin-5-one,
2-amino-7-(4-fluoro-2-pyrimidin-5-yl-phenyl)-4-methyl-6-(2-methyl-2-morpholin-4-yl-propyl)-7,8-dihydro-6H-pyrido[4,3-d]pyrimidin-5-one,
2-amino-7-[4-fluoro-2-(2-methoxy-pyridin-3-yl)-phenyl]-4-methyl-6-(2-methyl-2-morpholin-4-yl-propyl)-7,8-dihydro-6H-pyrido[4,3-d]pyrimidin-5-one,
2-amino-7-(5-fluoro-3'-methoxy-biphenyl-2-yl)-4-methyl-6-(2-methyl-2-morpholin-4-yl-propyl)-7,8-dihydro-6H-pyrido[4,3-d]pyrimid in-5-one,
(R)-2-amino-7-[4-fluoro-2-(4-methoxy-5-methyl-pyrimidin-2-yl)-phenyl]-4-methyl-7,8-dihydro-6H-pyrido[4,3-d]pyrimidin-5-one,
2-amino-7-(4-fluoro-2-furan-3-yl-phenyl)-4-methyl-7,8-dihydro-6H-pyrido[4,3-d]pyrimidin-5-one, or
a pharmaceutically acceptable salt or prodrug thereof.

8. The use of claim 7, wherein the Hsp90 inhibitor is (R)-2-amino-7-[4-fluoro-2-(6-methoxy-pyridin-2-yl)-phenyl]-4-methyl-7,8-dihydro-6H-pyrido[4,3-d]pyrimidin-5-one, or a pharmaceutically acceptable salt or prodrug thereof.

9. The use of claim 8, wherein the Hsp90 inhibitor is
(R)-2-amino-7-[2-(2-fluoro-pyridin-3-yl)-phenyl]-4-methyl-7,8-dihydro-6H-pyrido[4,3-d]pyrimidin-5-one,
(S)-2-amino-6-benzyl-7-[4-fluoro-2-(2-fluoro-pyridin-3-yl)-phenyl]-4-methyl-7,8-dihydro-6H-pyrido[4,3-d]pyrimidin-5-one,
(R)-2-amino-7-[4-fluoro-2-(2-fluoro-pyridin-3-yl)-phenyl]-4-methyl-7,8-dihydro-6H-pyrido[4,3-d]pyrimidin-5-one,
(R)-2-amino-7-(2-bromo-4-fluoro-phenyl)-6-[(S)-1-(4-methoxy-phenyl)-ethyl]-4-methyl-7,8-dihydro-6H-pyrido[4,3-d]pyrimidin-5-one,
(R)-2-amino-7-[2-(6-methoxy-pyridin-2-yl)-phenyl]-4-methyl-7,8-dihydro-6H-pyrido[4,3-d]pyrimidin-5-one,
(R)-2-amino-7-[4-fluoro-2-(6-methoxy-pyridin-2-yl)-phenyl]-4-methyl-7,8-dihydro-6H-pyrido[4,3-d]pyrimidin-5-one,
2-amino-7-[4-fluoro-2-(6-methoxy-pyridin-2-yl)-phenyl]-4,6-dimethyl-7,8-dihydro-6H-pyrido[4,3-d]pyrimidin-5-one,
2-amino-7-[4-fluoro-2-(2-fluoro-pyridin-3-yl)-phenyl]-4,6-dimethyl-7,8-dihydro-6H-pyrido[4,3-d]pyrimidin-5-one,
2-amino-7-[4-fluoro-2-(6-methoxypyridin-2-yl)phenyl]-4-methyl-7,8-dihydropyrido[4,3-*d*]pyrimidin-5(6*H*)-one,
2-amino-7-[2-(6-methoxy-pyrazin-2-yl)-phenyl]-4-methyl-7,8-dihydro-6H-pyrido[4,3-d]pyrimidin-5-one,
(R)-2-amino-7-[4-fluoro-2-(6-methoxy-pyrazin-2-yl)-phenyl]-4-methyl-7,8-dihydro-6H-pyrido[4,3-d]pyrimidin-5-one,
2-amino-7-[4-fluoro-2-(6-methoxy-pyrazin-2-yl)-phenyl]-4,6-dimethyl-7,8-dihydro-6H-pyrido[4,3-d]pyrimidin-5-one,
2-amino-7-[2-(2-methoxy-pyridin-3-yl)-phenyl]-4-methyl-7,8-dihydro-6H-pyrido[4,3-d]pyrimidin-5-one,
2-amino-7-(5,2'-difluoro-biphenyl-2-yl)-4-methyl-7,8-dihydro-6H-pyrido[4,3-d]pyrimidin-5-one,
2-amino-7-(5-fluoro-2'-trifluoromethoxy-biphenyl-2-yl)-4-methyl-7,8-dihydro-6H-pyrido[4,3-d]pyrimidin-5-one,
2-amino-7-[2-(2-chloro-pyridin-3-yl)-4-fluoro-phenyl]-4-methyl-7,8-dihydro-6H-pyrido[4,3-d]pyrimidin-5-one,
2-amino-7-[4-fluoro-2-(6-fluoro-pyridin-3-yl)-phenyl]-4-methyl-7,8-dihydro-6H-pyrido[4,3-d]pyrimidin-5-one,
2-amino-7-(4-fluoro-2-isoquinolin-4-yl-phenyl)-4-methyl-7,8-dihydro-6H-pyrido[4,3-d]pyrimidin-5-one,
2-amino-7-(5,3'-difluoro-biphenyl-2-yl)-4-methyl-7,8-dihydro-6H-pyrido[4,3-d]pyrimidin-5-one,
2-amino-7-[2-(4-chloro-pyridin-3-yl)-4-fluoro-phenyl]-4-methyl-7,8-dihydro-6H-pyrido[4,3-d]pyrimidin-5-one,
2-amino-7-(5,2'-difluoro-3'-methoxy-biphenyl-2-yl)-4-methyl-7,8-dihydro-6H-pyrido[4,3-d]pyrimidin-5-one,
2-amino-7-(5,4'-difluoro-2'-methyl-biphenyl-2-yl)-4-methyl-7,8-dihydro-6H-pyrido[4,3-d]pyrimidin-5-one,
2-amino-7-(5-fluoro-2'-methoxy-biphenyl-2-yl)-4-methyl-7,8-dihydro-6H-pyrido[4,3-d]pyrimidin-5-one,
2-amino-7-(4-fluoro-2-pyrimidin-5-yl-phenyl)-4-methyl-7,8-dihydro-6H-pyrido[4,3-d]pyrimidin-5-one,
2-amino-7-[4-fluoro-2-(2-methoxy-pyridin-3-yl)-phenyl]-4-methyl-7,8-dihydro-6H-pyrido[4,3-d]pyrimidin-5-one,
2-amino-7-(5-fluoro-3'-methoxy-biphenyl-2-yl)-4-methyl-7,8-dihydro-6H-pyrido[4,3-d]pyrimidin-5-one,
(R)-2-amino-6-(3-amino-propyl)-7-[4-fluoro-2-(6-methoxy-pyridin-2-yl)-phenyl]-4-methyl-7,8-dihydro-6H-pyrido[4,3-d]pyrimidin-5-one,
2-amino-7-(4-fluoro-2-pyridin-3-yl-phenyl)-4-methyl-7,8-dihydro-6H-pyrido[4,3-d]pyrimidin-5-one,
2-amino-7-(5,2'-difluoro-4'-methyl-biphenyl-2-yl)-4-methyl-7,8-dihydro-6H-pyrido[4,3-d]pyrimidin-5-one,
2-amino-7-[4-fluoro-2-(1-methyl-1H-pyrazol-4-yl)-phenyl]-4-methyl-7,8-dihydro-6H-pyrido[4,3-d]pyrimidin-5-one,
2-amino-7-[4-fl uoro-2-(1H-pyrazol-4-yl)-phenyl]-4-methyl-7,8-dihydro-6H-pyrido[4,3-d]pyrimidin-5-one,
2-amino-4-methyl-7-(5,2',3'-trifluoro-biphenyl-2-yl)-7,8-dihydro-6H-pyrido[4,3-d]pyrimidin-5-one,
2-amino-7-(2-bromo-4-fl uoro-phenyl)-4-methyl-6-(2-methyl-2-morpholin-4-yl-propyl)-7,8-dihydro-6H-pyrido[4,3-d]pyrimidin-5-one,
2-amino-7-(3'-dimethylamino-5-fluoro-biphenyl-2-yl)-4-methyl-7,8-dihydro-6H-pyrido[4,3-d]pyrimidin-5-one,
2-amino-7-[2-(2 ,4-dimethoxy-pyrimidin-5-yl)-4-fluoro-phenyl]-4-methyl-7,8-dihydro-6H-pyrido[4,3-d]pyrimidin-5-one,
2-amino-7-[4-fluoro-2-(5-methoxy-pyridin-3-yl)-phenyl]-4-methyl-7,8-dihydro-6H-pyrido[4,3-d]pyrimidin-5-one,
2-amino-7-(4-fl uoro-2-pyrimidin-5-yl-phenyl)-4-methyl-6-(2-methyl-2-morpholin-4-yl-propyl)-7,8-dihydro-6H-pyrido[4,3-d]pyrimidin-5-one,
2-amino-7-[4-fluoro-2-(2-methoxy-pyridin-3-yl)-phenyl]-4-methyl-6-(2-methyl-2-morpholin-4-yl-propyl)-7,8-dihydro-6H-pyrido[4,3-d]pyrimidin-5-one,
2-amino-7-(5-fluoro-3'-methoxy-biphenyl-2-yl)-4-methyl-6-(2-methyl-2-morpholin-4-yl-propyl)-7,8-dihydro-6H-pyrido[4,3-d]pyrimidin-5-one,
(R)-2-amino-7-[4-fluoro-2-(4-methoxy-5-methyl-pyrimidin-2-yl)-phenyl]-4-methyl-7,8-dihydro-6H-pyrido[4,3-d]pyrimidin-5-one,
2-amino-7-(4-fluoro-2-furan-3-yl-phenyl)-4-methyl-7,8-dihydro-6H-pyrido[4,3-d]pyrimidin-5-one, or
a pharmaceutically acceptable salt or prodrug thereof.

10. The use of claim 9, wherein the Hsp90 inhibitor is (R)-2-amino-7-[4-fluoro-2-(6-methoxy-pyridin-2-yl)-phenyl]-4-methyl-7,8-dihydro-6H-pyrido[4,3-d]pyrimidin-5-one, or a pharmaceutically acceptable salt or prodrug thereof.
